(19) Europäisches Patentamt
European Patent Office
Office européen des brevets

(11) Veröffentlichungsnummer: **0 220 296 B1**

# (12) EUROPÄISCHE PATENTSCHRIFT

(45) Veröffentlichungstag der Patentschrift: **28.07.93**

(51) Int. Cl.$^5$: **C09K 19/34**, C09K 19/42

(21) Anmeldenummer: **86903293.8**

(22) Anmeldetag: **19.04.86**

(86) Internationale Anmeldenummer:
**PCT/EP86/00234**

(87) Internationale Veröffentlichungsnummer:
**WO 86/06401 (06.11.86 86/24)**

Die Akte enthält technische Angaben, die nach dem Eingang der Anmeldung eingereicht wurden und die nicht in dieser Patentschrift enthalten sind.

## (54) SMEKTISCHE FLÜSSIGKRISTALLINE PHASEN.

(30) Priorität: **27.04.85 DE 3515374**

(43) Veröffentlichungstag der Anmeldung:
**06.05.87 Patentblatt 87/19**

(45) Bekanntmachung des Hinweises auf die Patenterteilung:
**28.07.93 Patentblatt 93/30**

(84) Benannte Vertragsstaaten:
**AT BE CH DE FR GB IT LI NL SE**

(56) Entgegenhaltungen:
**EP-A- 0 104 011**
**EP-A- 0 111 695**
**EP-A- 0 139 996**
**EP-A- 0 151 446**
**US-A- 4 391 730**

**See also references of WO8606401**

(73) Patentinhaber: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **HOPF, Reinhard**
**Wolfsgasse 3**
**W-6432 Heringen(DE)**
Erfinder: **SCHEUBLE, Bernhard**
**Am Grenzweg 18**
**W-6146 Alsbach(DE)**
Erfinder: **HITTICH, Reinhard**
**Am Kirchberg 11**
**W-6101 Modautal 1(DE)**
Erfinder: **KRAUSE, Joachim**
**Samuel-Morse-Strasse 14**
**W-6110 Dieburg(DE)**
Erfinder: **REIFFENRATH, Volker**
**Pfungstädterstrasse 31**
**W-6100 Darmstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **POETSCH, Eike**
**Am Buchwald 4**
**W-6109 Mühltal 6(DE)**
Erfinder: **GEELHAAR, Thomas**
**Kurt-Schumacher-Strasse 93**
**W-6500 Mainz(DE)**
Erfinder: **EIDENSCHINK, Rudolf**
**Kornblumenstrasse 1**
**W-6115 Münster(DE)**

## Beschreibung

Chirale getiltete smektische flüssigkristalline Phase, dadurch gekennzeichnet, daß sie mindestens drei Verbindungen der Formel I enthält,

$R^1$-$A^1$-$A^2$-$R^2$ I

worin

$R^1$ und $R^2$ jeweils Alkyl mit 1-15 C-Atomen, worin auch eine oder zwei nicht benachbarte $CH_2$-Gruppen durch -O-, -S-, -CO-, -O-CO-, -CO-O-, -CO-S-, -S-CO-, -CHHalogen-, -CHCN- und/oder -CH=CH- ersetzt sein können,

$A^1$ 1,4-Phenylen, trans-1,4-Cyclohexylen oder eine Einfachbindung, und

$A^2$

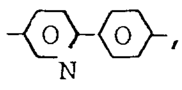, 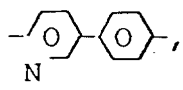, 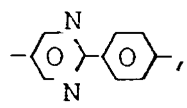, 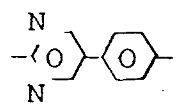

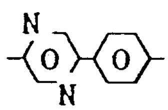, 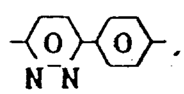, 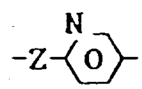, 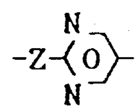,

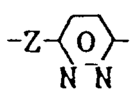 oder 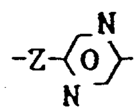

bedeutet,

wobei Z -O-CO-, -CO-O-, -S-CO-, -CO-S-, $-CH_2O-$, $-OCH_2-$ oder $-CH_2CH_2-$ bedeutet, enthält mit den Maßgaben, daß

a) $A^2$

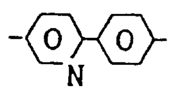, 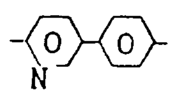, 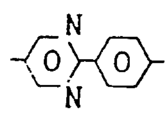, 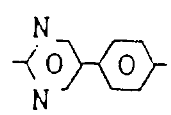

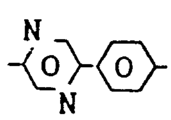 oder 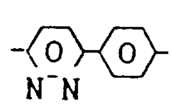

bedeutet, falls $A^1$ eine Einfachbindung ist, und

b) falls $A^1$ eine Einfachbindung ist und $A^2$

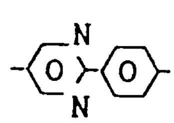

bedeutet,
für die Vertragsstaaten CH, DE, FR, GB, LI Phasen enthaltend Verbindungen der Formel

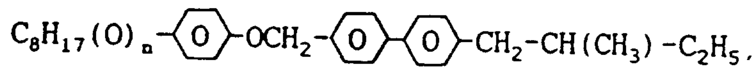

worin n 0 oder 1 ist, ausgenommen sind.

Chirale getiltete smektische flüssigkristalline Phasen mit ferroelektrischen Eigenschaften könnten hergestellt werden, in dem man Basis-Mischungen mit einer oder mehreren getilteten smektischen Phasen mit einem geeigneten chiralen Dotierstoff versetzt (L.A. Beresnev et al., Mol. Cryst. Liq. Cryst. 89, 327 (1982); H.R. Brand et al., J. Physique 44 (lett.), L-771 (1983). Solche Phasen können als Dielektrika für schnell schaltende Displays verwendet werden, die auf dem von Clark und Lagerwall beschriebenen Prinzip der SSFLC-Technologie (N.A. Clark und S.T. Lagerwall, Appl. Phys. Lett. 36, 899 (1980); USP 4,367,924) auf der Basis der ferroelektrischen Eigenschaften der chiral getilteten Phase beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1 - 2 µm) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiral getilteten smektischen Phasen (wie z.B. Sc*, jedoch auch $S_H^*$, $S_I^*$, $S_J^*$, $S_K^*$, $S_G^*$, $S_F^*$) ist deren geringe chemische, thermische und Photo-Stabilität. Eine weitere nachteilige Eigenschaft von Displays basierend auf derzeit verfügbaren chiral getilteten smektischen Mischungen ist, daß die Spontanpolarisation zu kleine Werte aufweist, so daß das Schaltzeitverhalten der Displays ungünstig beeinflußt wird und/oder der Pitch und/oder der Tilt und/oder die Viskosität der Phasen nicht den Anforderungen der Display-Technologie entspricht. Darüberhinaus ist meist der Temperaturbereich der ferroelektrischen Phasen zu klein und liegt überwiegend bei zu hohen Temperaturen.

Es wurde nun gefunden, daß die Verwendung von Verbindungen der Formel I als Komponenten chiral getilteter smektischer Mischungen die erwähnten Nachteile wesentlich vermindern kann. Die Verbindungen der Formel I sind somit als Komponenten chiral getilteter smektischer flüssigkristalliner Phasen vorzüglich geeignet. Insbesondere sind mit ihrer Hilfe chemisch besonders stabile chiral getiltete smektische flüssigkristalline Phasen mit günstigen ferroelektrischen Phasenbereichen, günstigen Werten für die Viskosität, insbesondere mit breiten Sc* Phasenbereichen, hervorragender Unterkühlbarkeit bis zu Temperaturen unter 0 °C ohne daß Kristallisation auftritt und für derartige Phasen hohen Werten für die spontane Polarisation herstellbar. P ist die spontane Polarisation in $nC/cm^2$.

Von L.A. Beresnev et al. (Abstracts of 9th International Conference on Liquid Crystals, Bangalore, India, 6 - 10 December 1982 sowie Ferroelectrics, **1984**, Vol. 59, S. 1-10) wurden die Spontanpolarisationen von binären, chiralen getilteten smektischen flüssigkristallinen Phasen untersucht, die neben einer chiralen Verbindung eine achirale Verbindung aufweisen. Unter anderem wurden dabei auch binäre Mischungen untersucht, die als achirale Komponente 5-p-Octyl-2(4-p'-nonyloxy- bzw. p'-octyloxy-phenyl)-pyrimidin enthalten. Diese binären Mischungen weisen jedoch ein ungenügendes Tieftemperatur-Schaltverhalten auf.

In der EP-A-0 178 647, die einen Stand der Technik gemäß Art. 54 (3) (4) darstellt, werden ferroelektrische Mischungen beschrieben, die neben achiralen 5-n-Alkyl-2-(alkoxyphenyl)-pyrimidinen optisch aktive Verbindungen der Formel

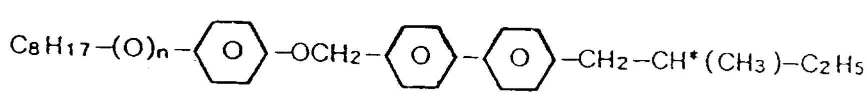

worin n 0 oder 1 bedeutet, enthalten. Diese Mischungen sind gemäß Maßgabe b) für die überlappenden Vertragsstaaten (CH, DE, FR, GB, LI) ausgenommen.

Die Verbindungen der Formel I besitzen einen breiten Anwendungsbereich. In Abhängigkeit von der Auswahl der Substituenten können diese Verbindungen als Basismaterialien dienen, aus denen flüssigkristalline smektische Phasen zum überwiegenden Teil zusammengesetzt sind; es können aber auch Verbindungen der Formel I flüssigkristallinen Basismaterialien aus anderen Verbindungklassen zugesetzt werden, um beispielsweise die dielektrische und/oder optische Anisotropie und/oder die Viskosität und/oder die spontane Polarisation und/oder den Phasenbereiche und/oder der Tiltwinkel und/oder den Pitch eines solchen Dielektrikums zu variieren.

Gegenstand der Erfindung sind chiral getiltete smektische flüssigkristalline Phasen mit einem Gehalt an mindestens drei Verbindungen der Formel I sowie Flüssigkristallanzeigeelemente, insbesondere ferroelektrische elektrooptische Anzeigeelemente, die derartige Phasen enthalten.

Die erfindungsgemäßen Phasen enthalten mindestens drei, insbesondere mindestens fünf Verbindungen der Formel I. Besonders bevorzugt sind erfindungsgemäße chiral getiltete smektische flüssigkristalline Phasen, deren achirale Basismischung neben Verbindungen der Formel I mindestens eine andere Komponente mit negativer oder betragsmäßig kleiner positiver dielektrischer Anisotropie enthält. Diese weiteren Komponente(n) der achiralen Basismischung können 1 bis 50 %, vorzugsweise 10 bis 25 %, der Basismischung ausmachen. Als weitere Komponenten mit betragsmäßig kleiner positiver oder negativer dielektrischer Anisotropie eignen sich Verbindungen der Formel II, welche die Verbindungen der Teilformeln IIa bis IIi umfaßt:

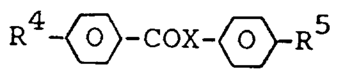 IIa

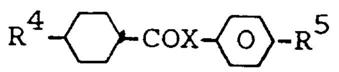 IIb

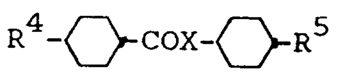 IIc

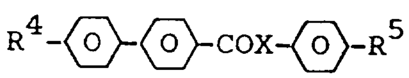 IId

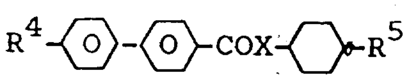 IIe

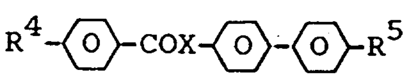 IIf

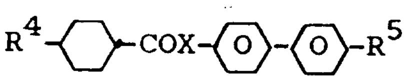 IIg

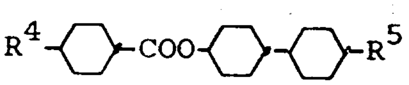 IIh

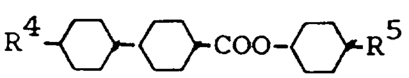 IIi

$R^4$ und $R^5$ sind jeweils vorzugsweise geradkettiges Alkyl, Alkoxy, Alkanoyloxy oder Alkoxycarbonyl mit jeweils 3 bis 12 C-Atomen. X ist vorzugsweise O. In den Verbindungen der Formeln IIa, IIb, IId, IIe, IIf und IIg kann auch eine 1,4-Phenylengruppe lateral durch Halogen oder CN, insbesondere bevorzugt durch Fluor, substituiert sein.

Besonders bevorzugt sind die Verbindungen der Teilformeln IIa, IIb, IId und IIf, worin $R^4$ und $R^5$ jeweils geradkettiges Alkyl oder Alkoxy mit jeweils 5 bis 10 C-Atomen bedeutet.

Besonders bevorzugte Einzelverbindungen sind in der folgenden Tabelle angegeben:

| Formel | $R^4$ | $R^5$ | X |
|---|---|---|---|
| IIa | n-Decyloxy | n-Heptyloxy | O |
| IIa | n-Hexyloxy | n-Decyloxy | O |
| IIa | n-Octyloxy | n-Heptyl | O |
| IIa | n-Octyloxy | n-Pentyl | O |
| IIa | n-Decyloxy | n-Heptyl | O |
| IIa | n-Decyloxy | n-Pentyl | O |
| IIf | n-Pentyl | n-Pentyl | O |
| IIf | n-Pentyl | n-Hexyl | O |

Die Verbindungen der Teilformeln IIc, IIh und IIi eignen sich als Zusätze zur Schmelzpunktserniedrigung und werden normalerweise den Basismischungen mit nicht mehr als 5 %, vorzugsweise 1 bis 3 %, zugesetzt. $R^4$ und $R^5$ bedeuten in den Verbindungen der Teilformeln IIc, IIh und IIi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist diejenige der Formel

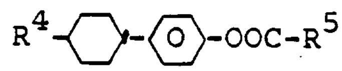

worin $R^4$ und $R^5$ die für IIc, IIh und IIi angegebene bevorzugte Bedeutung haben.

Als weitere Komponenten mit negativer dielektrischer Anisotropie eignen sich weiterhin Verbindungen enthaltend das Strukturelement A, B oder C.

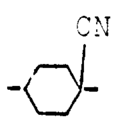

A

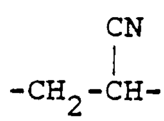

B

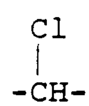

C

Bevorzugte Verbindungen dieser Art entsprechen den Formeln IIIa, IIIb und IIIc:

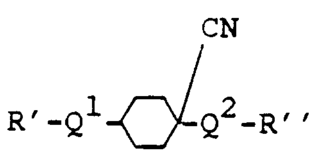

IIIa

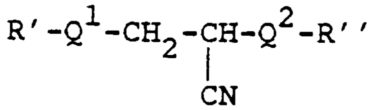

IIIb

$R'-Q^3-Q^4-R'''$ IIIc

R' und R'' bedeuten jeweils vorzugsweise geradkettige Alkyl- oder Alkoxy-Gruppen mit jeweils 2 bis 10 C-Atomen. $Q^1$ und $Q^2$ bedeuten jeweils 1,4-Phenylen, trans-1,4-Cyclohexylen, 4,4'-Biphenylyl, 4-(trans-4-Cyclohexyl)-phenyl, trans,trans-4,4'-Bicyclohexyl oder eine der Gruppen $Q^1$ und $Q^2$ auch eine Einfachbin-

dung.

$Q^3$ und $Q^4$ bedeuten jeweils 1,4-Phenylen, 4,4'-Biphenylyl oder trans-1,4-Cyclohexylen. Eine der Gruppen $Q^3$ und $Q^4$ kann auch 1,4-Phenylen bedeuten, worin mindestens eine CH-Gruppe durch N ersetzt ist. R''' ist ein optisch aktiver Rest mit einem asymmetrischen Kohlenstoffatom der Struktur

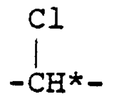 oder 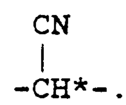.

Besonders bevorzugte Verbindungen der Formel IIIc sind diejenigen der Formel IIIc':

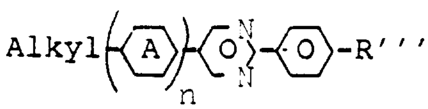

IIIc'

worin A 1,4-Phenylen oder trans-1,4-Cyclohexylen und n 0 oder 1 bedeutet.

Die Verbindungen der Formel I umfassen zweikernige und dreikernige Materialien. Von den zweikernigen, welche bevorzugt sind, sind diejenigen bevorzugt, worin $R^1$ n-Alkyl mit 7 bis 10, insbesondere 7 bis 9, C-Atome bedeutet. Verbindungen der Formel I mit $R^1$ = n-Heptyl oder n-Octyl verleihen den erfindungsgemäßen Phasen ein gutes Tieftemperaturverhalten, während die entsprechenden Verbindungen mit $R^1$ = n-Nonyl die $S_A/S_C$-Umwandlungstemperatur der erfindungsgemäßen Phasen zu erhöhen vermögen.

Bevorzugte erfindungsgemäße Phasen enthalten mindestens eine Verbindung der Formel I, worin $R^1$ n-Nonyl bedeutet und mindestens eine Verbindung der Formel I, worin $R^1$ n-Heptyl oder n-Octyl bedeutet. Besonders bevorzugt sind erfindungsgemäße Phasen enthaltend Verbindungen der Formel I, worin $R^1$ n-Heptyl, n-Octyl und n-Nonyl bedeutet. $R^2$ ist in den zweikernigen Verbindungen der Formel I vorzugsweise n-Alkoxy mit 6 bis 12, insbesondere mit 7 bis 10, C-Atomen. Vorzugsweise enthalten die erfindungsgemäßen Phasen mindestens eine Verbindung der Formel I, worin $R^2$ n-Hexyloxy, n-Heptyloxy oder n-Octyloxy (vorzugsweise n-Heptyloxy oder n-Octyloxy) bedeutet und mindestens eine Verbindung der Formel I, worin $R^2$ n-Nonyloxy oder n-Decyloxy bedeutet. Die Summe der C-Atome in den Gruppen $R^1$ und $R^2$ der bevorzugten zweikernigen Verbindungen der Formel I ist vorzugsweise 15 oder höher, besonders bevorzugt im Bereich 15 bis 20. Besonders bevorzugte Einzelverbindungen der Formel I sind in der folgenden Tabelle aufgeführt:

| $R^1$ | $R^2$ | $A^1$ |
|---|---|---|
| n-Nonyl | n-Nonyloxy | - |
| n-Nonyl | n-Hexyloxy | - |
| n-Octyl | n-Decyloxy | - |
| n-Octyl | n-Octyloxy | - |
| n-Octyl | n-Heptyloxy | - |
| n-Heptyl | n-Decyloxy | - |
| n-Heptyl | n-Nonyloxy | - |
| n-Pentyloxy | n-Octyl | 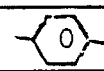 |
| n-Hexyloxy | n-Hexyl | 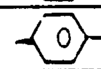 |
| n-Hexyloxy | n-Pentyl | 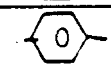 |
| n-Pentyloxy | n-Nonyl | 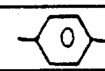 |
| n-Octyloxy | n-Octyl | 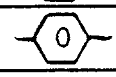 |

Weiterhin bevorzugt sind Verbindungen der Formel I worin $R^1$ n-Alkyl mit 7 bis 10 C-Atomen bedeutet und $R^2$ n-Alkanoyloxy, n-Alkoxycarbonyl oder n-Alkylthio mit jeweils 5 bis 10 C-Atomen ist.

Die erfindungsgemäßen Phasen enthalten vorzugsweise mindestens eine dreikernige Verbindung der Formel I und/oder eine Verbindung der Formel I, worin $R^2$ n-Alkylthio bedeutet. Diese Phasen zeichnen sich durch besonders hohe $S_C/S_A$-Umwandlungstemperaturen aus.

Ferner bevorzugt sind jedoch erfindungsgemäße Phasen enthaltend lediglich Verbindungen der Formel I, worin $A^1$ eine Einfachbindung bedeutet. Diese Phasen zeichnen sich durch ein besonders günstiges Tieftemperaturverhalten und besonders niedrige Viskositätswerte aus. Ferner bevorzugt sind erfindungsgemäße Phasen enthaltend Verbindungen der Formel I, worin mindestens eine Gruppe $R^1$ oder $R^2$ einen verzweigtkettigen Alkyl- oder Alkoxyrest darstellt. Diese Phasen zeigen ebenfalls ein günstiges Tieftemperaturverhalten.

$R^1$ und $R^2$ sind jeweils unabhängig voneinander vorzugsweise Alkyl, Alkoxy, Alkanoyl, Alkanoyloxy, Alkoxycarbonyl oder Alkoxycarbonyloxy mit jeweils vorzugsweise 5 bis 12, insbesondere 6 bis 10 C-Atomen. Besonders bevorzugt sind Alkyl und Alkoxy. Vorzugsweise ist eine der Gruppen $R^1$ und $R^2$ Alkyl. Eine besonders bevorzugte Kombination ist $R^1$ = Alkyl und $R^2$ = Alkoxy und ferner $R^1$ = Alkoxy und $R^2$ = Alkoxy. Besonders bevorzugt sind $R^1$- und $R^2$-Gruppen mit geradkettigem Alkylrest.

Weiterhin bevorzugt sind erfindungsgemäße Phasen enthaltend mindestens eine zweikernige Verbindung der Formel I worin $R^1$ n-Alkyl mit 7 bis 10 C-Atomen und $R^2$ $-CH_2O-(CH_2)_p-CH_3$ oder $-O-(CH_2)_q-O-(CH_2)_r-CH_3$ bedeutet.

p ist vorzugsweise 4 bis 10, insbesondere 5 bis 9. q ist vorzugsweise 1 oder 2, insbesondere bevorzugt 2.

r ist 4 bis 10, insbesondere bevorzugt 5 bis 9.

q kann auch > 2, z.B. 3 bis 5 sein.

$A^2$ bedeutet vorzugsweise

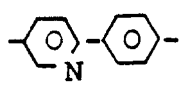

oder

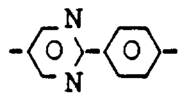

insbesondere bevorzugt

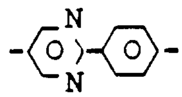

$A^1$ ist vorzugsweise eine Einfachbindung oder im Falle
$A^2$ =

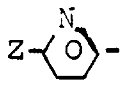, 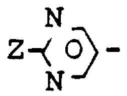, 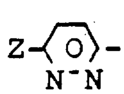 oder 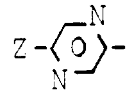

1,4-Phenylen.
Z ist vorzugsweise -CO-O-, -O-CO- oder $-CH_2CH_2-$.
Weitere bevorzugte Bedeutungen für $A^2$ sind

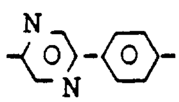 und 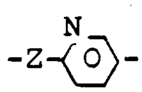.

Ferner bevorzugt sind erfindungsgemäße Phasen enthaltend mindestens eine Verbindung der Formel

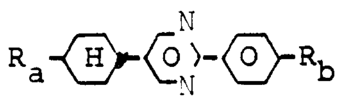 oder 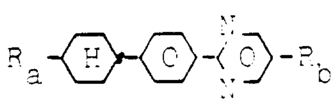

worin $R_a$ Alkyl mit 3 bis 12 C-Atomen und $R_b$ Alkyl oder Alkoxy mit jeweils 5 bis 12 C-Atomen bedeutet. $R_a$ und $R_b$ enthalten zusammen mindestens 15 C-Atome, vorzugsweise mindestens 17 C-Atome.
Besonders bevorzugt ist eine chirale getiltete smektische flüssigkristalline Phase mit mindestens einer Verbindung der Formel I nach Anspruch 1, dadurch gekennzeichnet, daß sie eine flüssigkristalline Komponente mit negativer dielektrischer Anisotropie enthält, insbesondere eine erfindungsgemäße Phase dadurch gekennzeichnet, daß sie als Komponente mit negativer dielektrischer Anisotropie mindestens eine das strukturelement A, B oder C aufweisende Verbindung enthält.

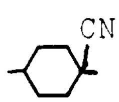
A

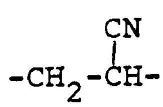
B

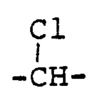
C

Besonders bevorzugt ist ferner eine erfindungsgemäße Phase, dadurch gekennzeichnet, daß sie mindestens eine Verbindung der Formel II enthält,

$R^4-A^1-COX-A^2-R^5$ II

worin

$R^4$ und $R^5$ jeweils Alkyl mit 1-15 C-Atomen, worin auch eine oder zwei nicht benachbarte $CH_2$-Gruppen durch -O-, -CO-, -O-CO-, -CO-O-und/oder -CH=CH- ersetzt sein können,

X O oder S,

und

$A^1$ und $A^2$ jeweils 1,4-Phenylen oder trans-1,4-Cyclohexylen bedeuten, eine der Gruppen $A^1$ und $A^2$ gegebenenfalls auch 4,4'-Biphenylyl oder trans,trans-4,4'-Bicyclohexyl, bedeuten.

Weiterhin bevorzugt sind erfindungsgemäße ferroelektrische Phasen enthaltend mindestens eine Verbindung der Formel V

$R^1$-$Q^1$-A-$(Q^2)_q$-$R^2$ V

worin

$R^1$ und $R^2$ jeweils unabhängig voneinander eine geradkettige Alkylgruppe mit 1 bis 15 C-Atomen, worin auch eine oder mehrere nicht benachbarte $CH_2$-Gruppen durch -O-, -S-, -CO-, $CHCH_3$-O-, -$CHCH_3$-, -CH-Halogen-, CHCN-, -O-CO-, -O-COO-, -CO-O- und/oder -CH=CH-ersetzt sein können,

A

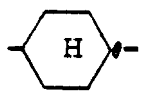,
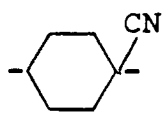,
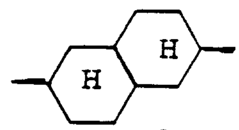,

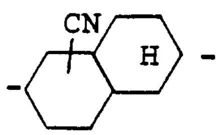,
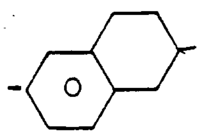,
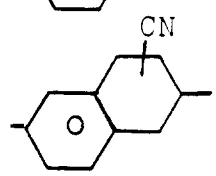,

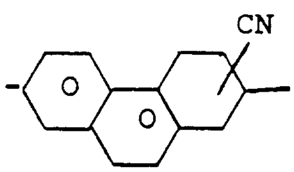,
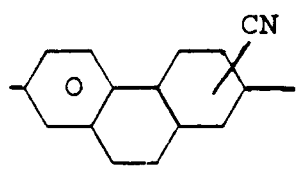,

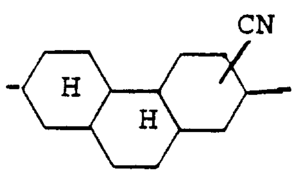,
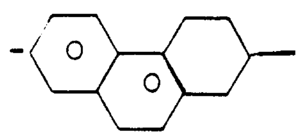,

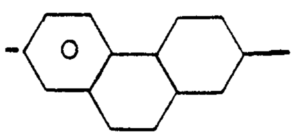,
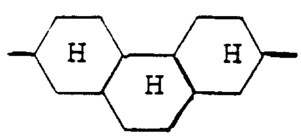,

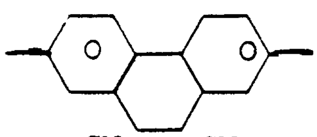,
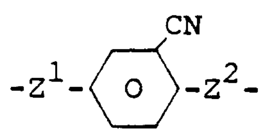 oder

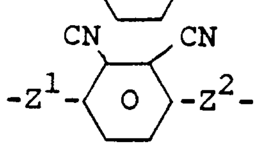

q — 0 oder 1,

$Q^1$ und $Q^2$ — jeweils unabhängig voneinander, $-(A°-Z°)_p-$,wobei

A° — unsubstituiertes oder ein- oder mehrfach durch Halogenatome, $CH_3$- und/oder Nitril-Gruppen substituiertes 1,4-Cyclohexylen, worin auch eine oder zwei nicht benachbarte $CH_2$-Gruppen durch -O- und/oder -S- und/oder eine

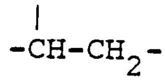

Gruppierung durch

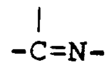

ersetzt sein können (Cy), oder unsubstituiertes oder ein- oder mehrfach durch Halogenatome, $CH_3$- und/oder Nitril-Gruppen substituiertes 1,4-Phenylen, worin auch eine oder mehrere CH-Gruppen durch N ersetzt sein können (Ph) bedeutet, einer der Reste A° auch 2,6-Naphthylen (Na) oder Tetrahydro-2,6-naphthylen (4H-Na), gegebenenfalls durch Halogen oder CN substituiert,

Z°, $Z^1$ und $Z^2$ — jeweils unabhängig voneinander -CO-O-, -O-CO--$CH_2$O-,O$CH_2$-, -$CH_2CH_2$-, -CHC$NCH_2$-, -$CH_2$-CHCN- oder eine Einfachbindung, und

p — 1, 2 oder 3, oder im Falle A = Tetra- oder Octahydrophenanthren auch 0 bedeutet, wobei Falle A =

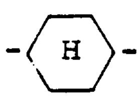 , 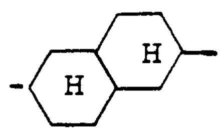 , 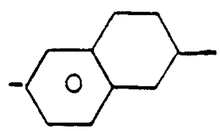 ,

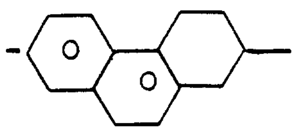 , 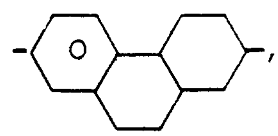 ,

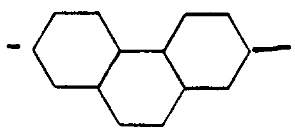 oder 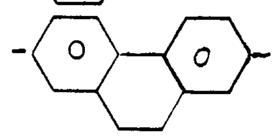

mindestens eine Gruppe Z° -CHCN$CH_2$- oder -$CH_2$CHCN-bedeutet und/oder in mindestens einer der Gruppen $R^1$ und $R^2$ mindestens eine $CH_2$-Gruppe durch -CHCN- ersetzt ist.

Die Verbindungen der Formel V können geradkettige oder verzweigte Flügelgruppen $R^1$ und/oder $R^2$ haben. Verbindungen mit verzweigten Flügelgruppen können in Form des Racemates oder als optisch aktive Verbindungen eingesetzt werden. Achirale Basismischungen aus Verbindungen der Formel V und ggf. weiteren achiralen Komponenten können mit chiralen Verbindungen der Formel I oder auch zusätzlich mit anderen chiralen Verbindungen dotiert werden, um chiral getiltete smektische Phasen zu erhalten.

Besonders bevorzugte kleinere Gruppen von Verbindungen sind diejenigen der Formeln V1 bis V18:

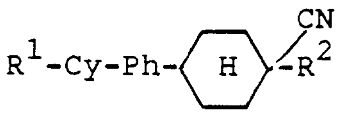

V1

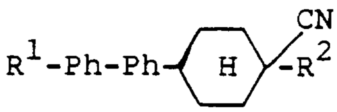

V2

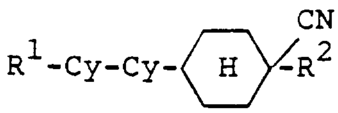

V3

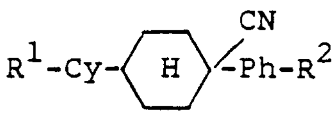

V4

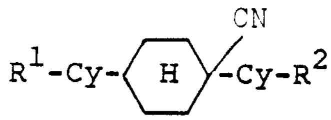

V5

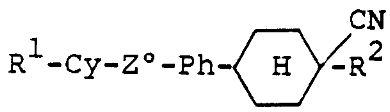

V6

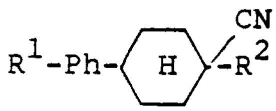

V7

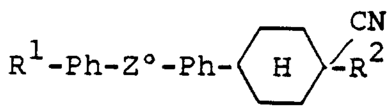

V8

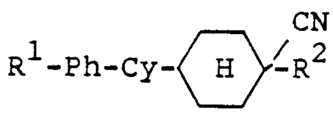

V9

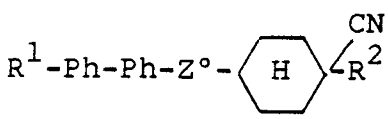

V10

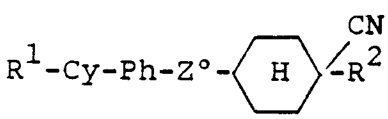

V11

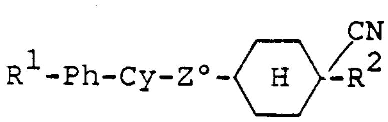

V12

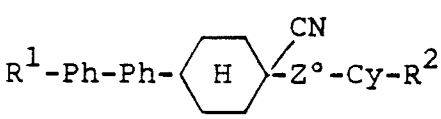

V13

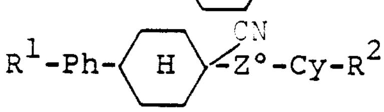

V14

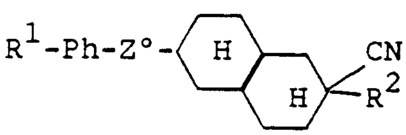

V15

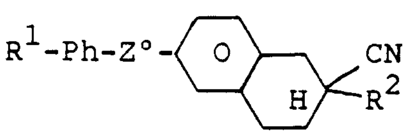

V16

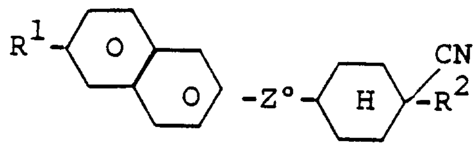

V17

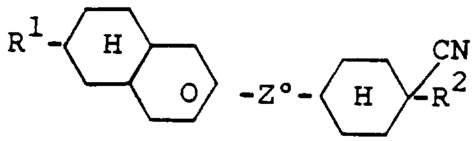

V18

Eine weitere besonders bevorzugte kleinere Gruppe von Verbindungen sind diejenigen der Formeln V19 bis V22:

$R^1$-$A^{\circ}$-Cy-$(CH_2)_r$-CHCN-$C_sH_{2s+1}$ V19

$R^1$-$A^{\circ}$-$A^{\circ}$-Cy-$(CH_2)_r$-CHCN-$C_sH_{2s+1}$ V20

$R^1$-$A^{\circ}$-$A^{\circ}$-CHCN-$CH_2$-Cy-$R^2$ V21

$R^1$-$A^{\circ}$-$A^{\circ}$-$CH_2$-CHCN-Cy-$R^2$ V22

worin r 0, 1, 2 oder 3 bedeutet und (r + s)1 bis 14 ist.

Verbindungen der Formel I, die keine $S_c$-Phasen aufweisen, eignen sich ebenfalls als Komponenten erfindungsgemäßer smektischer Phasen.

Die erfindungsgemäßen Phasen können ferner auch Verbindungen der Formel

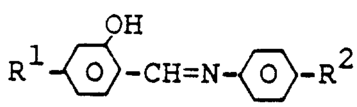

enthalten, worin $R^1$ und $R^2$ die bei Formel V angegebene Bedeutung haben.

Alle Komponenten der erfindungsgemäßen Phasen sind entweder bekannt oder in an sich bekannter Weise analog zu bekannten Verbindungen herstellbar.

Die Herstellung der erfindungsgemäßen Phasen erfolgt in an sich üblicher Weise. In den Regel werden die Komponenten ineinander gelöst, zweckmäßig bei erhöhter Temperatur.

Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können.

Die folgenden Beispiele sollen die Erfindung erläutern, ohne sie zu begrenzen. Vor- und nachstehend bedeuten Prozentangaben Gewichtsprozent; alle Temperaturen sind in Grad Celsius angegeben. Die Werte für die spontane Polarisation gelten für Raumtemperatur. Es bedeuten ferner: K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.

Beispiel 1

Eine flüssigkristalline Phase bestehend aus
25 % 2-p-Decyloxyphenyl-5-heptylpyrimidin,
25 % 2-p-Nonyloxyphenyl-5-nonylpyrimidin,
10 % 2-p-Hexyloxyphenyl-5-nonylpyrimidin,
10 % 2-p-Nonyloxyphenyl-5-heptylpyrimidin,
11 % 2-p-Octyloxyphenyl-5-heptylpyrimidin,
11 % 2-p-Heptyloxyphenyl-5-heptylpyrimidin und

8 % 2-p-Hexyloxyphenyl-5-heptylpyrimidin
ist unterkühlbar bis -1°, K/$S_C$ 11° und $S_C$/$S_A$ 49°.

Beispiel 2

Eine flüssigkristalline Phase bestehend aus
50 % 2-p-Nonyloxyphenyl-5-nonylpyrimidin,
7 % 2-p-Hexyloxyphenyl-5-nonylpyrimidin,
15 % 2-p-Decyloxyphenyl-5-heptylpyrimidin,
7 % 2-p-Nonyloxyphenyl-5-heptylpyrimidin,
7 % 2-p-Octyloxyphenyl-5-heptylpyrimidin,
7 % 2-p-Heptyloxyphenyl-5-heptylpyrimidin und
7 % 2-p-Hexyloxyphenyl-5-heptylpyrimidin
ist unterkühlbar bis 0°, K/$S_C$ 6° und $S_C$/$S_A$ 50°.

Beispiel 3

Eine flüssigkristalline Phase bestehend aus
25 % 2-p-Nonyloxyphenyl-5-nonylpyrimidin,
25 % 2-p-Decyloxyphenyl-5-heptylpyrimidin,
10 % 2-p-Nonyloxyphenyl-5-heptylpyrimidin,
11 % 2-p-Octyloxyphenyl-5-heptylpyrimidin,
11 % 2-p-Heptyloxyphenyl-5-heptylpyrimidin,
8 % 2-p-Hexyloxyphenyl-5-heptylpyrimidin,
10 % trans-4-Pentylcyclohexancarbonsäure-(p-hexyloxyphenylester)
ist bis -3° unterkühlbar ohne daß Kristallisation eintritt und hat $S_C$/$S_A$ 44°.

Beispiel 4

Eine flüssigkristalline Phase bestehend aus
25 % 2-p-Decyloxyphenyl-5-heptylpyrimidin,
20 % 2-p-Nonyloxyphenyl-5-heptylpyrimidin,
17 % 2-p-Octyloxyphenyl-5-heptylpyrimidin,
17 % 2-p-Heptyloxyphenyl-5-heptylpyrimidin,
11 % 2-p-Hexyloxyphenyl-5-heptylpyrimidin und
10 % trans-4-Pentylcyclohexancarbonsäure-(p-hexyloxyphenylester)
ist bis -6° unterkühlbar ohne daß Kristallisation eintritt und hat $S_C$/N 32°.

Beispiel 5

Eine flüssigkristalline Phase bestehend aus
22 % 2-p-Decyloxyphenyl-5-octylpyrimidin,
18 % 2-p-Nonyloxyphenyl-5-octylpyrimidin,
18 % 2-p-Octyloxyphenyl-5-octylpyrimidin,
18 % 2-p-Heptyloxyphenyl-5-octylpyrimidin,
13 % 2-p-Nonyloxyphenyl-5-heptylpyrimidin,
11 % trans-4-Pentylcyclohexancarbonsäure-(p-hexylphenylester)
ist bis -5° unterkühlbar und hat $S_C$/$S_A$ 46,1°, $S_A$N 57,6° und N/I 67,6°.

Beispiel 6

Eine flüssigkristalline Phase bestehend aus
20 % 2-p-Nonyloxyphenyl-5-nonylpyrimidin,
10 % 2-p-Nonyloxyphenyl-5-heptylpyrimidin,
18 % 2-p-Decyloxyphenyl-5-octylpyrimidin,
15 % 2-p-Octyloxyphenyl-5-octylpyrimidin,
12 % 2-p-Heptyloxyphenyl-5-octylpyrimidin und
25 % trans-4-Heptylcyclohexancarbonsäure-(p-hexyloxyphenylester)

ist bis 0° unterkühlbar und hat $S_C/S_A$ 36,9°, $S_A/N$ 61° und N/I 68,5°.

Beispiel 7

Eine flüssigkristalline Phase bestehend aus
12 % 2-p-Nonyloxyphenyl-5-heptylpyrimidin,
20 % 2-p-Decyloxyphenyl-5-octylpyrimidin,
16 % 2-p-Nonyloxyphenyl-5-octylpyrimidin,
16 % 2-p-Octyloxyphenyl-5-octylpyrimidin,
16 % 2-p-Heptyloxyphenyl-5-octylpyrimidin,
10% trans-4-Pentylcyclohexancarbonsäure-(p-hexyloxyphenylester) und
10% r-1-Cyan-1-(trans-4-pentylcyclohexyl)-trans-4-(trans-4-heptylcyclohexyl)-cyclohexan
hat $S_C/S_A$ 43,3°, $S_A/N$ 64,5° und N/I 75°.

Beispiel 8

Eine flüssigkristalline Phase bestehend aus
12% 2-p-Nonyloxyphenyl-5-heptylpyrimidin,
20% 2-p-Decyloxyphenyl-5-octylpyrimidin,
16% 2-p-Nonyloxyphenyl-5-octylpyrimidin,
16% 2-p-Octyloxyphenyl-5-octylpyrimidin,
16% 2-p-Heptyloxyphenyl-5-octylpyrimidin,
10% trans-4-Pentylcyclohexancarbonsäure-(p-hexyloxyphenylester) und
10% 1-Cyan-1-(trans-4-pentylcyclohexyl)-2-(trans-4-propylcyclohexyl)-ethan
hat $S_C/S_A$ 40,0°, $S_A/N$ 59,9° und N/I 72,5°.

Beispiel 9

Eine flüssigkristalline Phase bestehend aus
12% 2-p-Nonyloxyphenyl-5-heptylpyrimidin,
20% 2-p-Decyloxyphenyl-5-octylpyrimidin,
16% 2-p-Nonyloxyphenyl-5-octylpyrimidin,
16% 2-p-Octyloxyphenyl-5-octylpyrimidin,
16% 2-p-Heptyloxyphenyl-5-octylpyrimidin,
10% trans-4-Pentylcyclohexancarbonsäure-(p-hexyloxyphenylester) und
10% 1-Cyan-1-(trans-4-pentylcyclohexyl)-2-[trans-4-(trans-4-pentylcyclohexyl)-cyclohexyl]-ethan
hat $S_C/S_A$ 40,5°, $S_A/N$ 60,6° und N/I 72°.

Beispiel 10

Eine flüssigkristalline Phase bestehend aus
12 % 2-p-Nonyloxyphenyl-5-heptylpyrimidin,
20 % 2-p-Decyloxyphenyl-5-octylpyrimidin,
16 % 2-p-Nonyloxyphenyl-5-octylpyrimidin,
16 % 2-p-Octyloxyphenyl-5-octylpyrimidin,
16 % 2-p-Heptyloxyphenyl-5-octylpyrimidin,
10 % trans-4-Pentylcyclohexancarbonsäure-(p-hexyloxyphenylester),
3,5% trans-4-(trans-4-Pentylcyclohexyl)-1-hexanoyloxycyclohexan und
6,5% trans-4-(trans-4-Heptylcyclohexyl)-1-octanoyloxycyclohexan
hat $S_C/N$ 49,5° und N/I 68,7°.

Beispiel 11

Eine flüssigkristalline Phase bestehend aus
20 % 2-p-Nonyloxyphenyl-2-nonylpyrimidin,
10 % 2-p-Nonyloxyphenyl-2-heptylpyrimidin,
18 % 2-p-Decyloxyphenyl-2-octylpyrimidin,
15 % 2-p-Nonyloxyphenyl-2-octylpyrimidin,

15 % 2-p-Octyloxyphenyl-2-octylpyrimidin,
12 % 2-p-Heptyloxyphenyl-2-octylpyrimidin und
10 % r-1-Cyan-cis-4-(trans-4-butylcyclohexyl)-1-butyl-cyclohexan
hat $S_C/S_A$ 34,5°, $S_A$/N 56,6° und N/I 66,0°.

Beispiel 12

Eine flüssigkristalline Phase bestehend aus
20 % 2-p-Nonyloxyphenyl-5-nonylpyrimidin,
10 % 2-p-Nonyloxyphenyl-5-heptylpyrimidin,
18 % 2-p-Decyloxyphenyl-5-octylpyrimidin,
15 % 2-p-Nonyloxyphenyl-5-octylpyrimidin,
15 % 2-p-Octyloxyphenyl-5-octylpyrimidin,
12 % 2-p-Heptyloxyphenyl-5-octylpyrimidin und
10 % trans-4-Pentylcyclohexancarbonsäure-(p-hexyloxyphenylester)
wird mit verschiedenen Mengen der chiralen Verbindung p-(p-Hexyloxybenzoyloxy)-benzoesäure-(1-methylheptylester) dotiert. Die Phasenübergangstemperaturen sowie die Werte der spontanen Polarisation der somit erhaltenen Mischungen sind in der folgenden Tabelle angegeben. Die Werte für P gelten jeweils für 10° unterhalb des $Sc^*/S_A^*$-Übergangs.

Zugabe von X % des chiralen Dotierstoffes

| X | $S_C$* | $S_A$* | Ch | I | P |
|---|---|---|---|---|---|
| 0,95 | | 40,5 | 65,0 | 69,1 | 0,05 |
| 1,95 | | 45,5 | 65,1 | 68,9 | 0,12 |
| 3,11 | | 41,3 | 65,2 | 68,6 | 0,21 |
| 4,07 | | 37,3 | 65,0 | 67,7 | 0,33 |
| 4,93 | | 29,5 | 64,8 | 66,9 | 0,43 |
| 7,36 | | 19,7 | 64,7 | 66,1 | 0,60 |
| 9,96 | | - | 64,5 | 65,2 | |

Beispiel 13

Die flüssigkristalline Phase aus Beispiel 12 wird mit 1,09 (2,07 %) der chiralen Verbindung 4-(1-Methylpropoxy)-4'-cyanbiphenyl dotiert. Die dotierte Phase Zeigt $S_C^*/S_A^*$ 42,4 (26,5), $S_A^*$/Ch 65,7 (67,4) und Ch/I 68,9 (68,8).

Beispiel 14

Die flüssigkristalline Phase aus Beispiel 12 wird mit 1,10 (1,96) % der chiralen Verbindung 4-(1-Methylpropyl)-4'-cyanbiphenyl dotiert. Die dotierte Phase zeigt $S_C^*$ /$S_A^*$ 42,9 (26,9).

Beispiel 15

Die flüssigkristalline Phase aus Beispiel 12 wird mit verschiedenen Mengen der chiralen Verbindung p-(p-2-Methylbutylphenyl)-benzoesäure-(p-hexylphenylester) dotiert. Die Phasenübergangstemperaturen der somit erhaltenen Mischungen sind in der folgenden Tabelle angegeben:

Zugabe von X % des chiralen Dotierstoffes

| X | $S_C$* | $S_A$* | Ch | I |
|---|---|---|---|---|
| 1,00 | 52,3 | 65,2 | 69,7 | |
| 2,00 | 52,6 | 64,8 | 70,3 | |
| 3,02 | 52,8 | 65,0 | 70,2 | |
| 4,27 | 53,2 | 64,8 | 70,6 | |
| 6,15 | 54,7 | 64,2 | 70,4 | |
| 10,12 | 55,6 | 62,5 | 73,9 | |
| 19,97 | 56,9 | - | 80,4 | |
| 30,30 | 57,1 | - | | |
| 40,46 | 54,3 | - | | |
| 50,00 | 55,0 | - | | |

Beispiel 16

Die flüssigkristalline Phase aus Beispiel 12 wird mit verschiedenen Mengen der chiralen Verbindung p-(p-Octylphenyl)-benzoesäure-(p-2-methylbutylphenylester) dotiert. Die Phasenübergangstemperaturen der somit erhaltenen Mischungen sind in der folgenden Tabelle angegeben:

Zugabe von X % des chiralen Dotierstoffes

| X | $S_C$* | $S_A$* | Ch | I |
|---|---|---|---|---|
| 1,00 | 51,5 | 65,5 | 70,0 | |
| 2,15 | 50,6 | 66,0 | 70,4 | |
| 2,94 | 49,6 | 66,3 | 70,7 | |
| 4,06 | 48,8 | 66,6 | 70,9 | |
| 5,61 | 47,4 | 67,5 | 71,6 | |
| 6,33 | 46,4 | 68,3 | 72,3 | |
| 8,01 | 44,5 | 70,3 | 72,9 | |
| 10,21 | 42,3 | 71,0 | 73,8 | |
| 15,24 | 32,2 | 72,8 | 74,8 | |
| 20,79 | < 0 | | | |

Beispiel 17

Eine flüssigkristalline Phase bestehend aus
13 % 2-p-Nonyloxyphenyl-5-heptylpyrimidin,
22 % 2-p-Decyloxyphenyl-5-octylpyrimidin,
18 % 2-p-Nonyloxyphenyl-5-octylpyrimidin,
18 % 2-p-Octyloxyphenyl-5-octylpyrimidin,
18 % 2-p-Heptyloxyphenyl-5-octylpyrimidin und
11 % trans-4-Pentylcyclohexancarbonsäure-(p-hexyloxyphenylester)
wird mit verschiedenen Mengen der chiralen Verbindung p-(p-2-Methylbutylphenyl)-benzoesäure-(p-hexylphenylester) dotiert. Die Phasenübergangstemperaturen der somit erhaltenen Mischungen sind in der folgenden Tabelle angegeben:

| Zugabe von X % des chiralen Dotierstoffes X | $S_C$* | $S_A$* | Ch | I |
|---|---|---|---|---|
| 0,5* | 44,9 | 55,4 | 56,8 | |
| 2,0* | 47,0 | 57,1 | 68,4 | |
| 5,0* | 47,5 | 55,4 | 70,1 | |
| 10,0 | 50,5 | 58,3 | 70,5 | |

* Diese Mischungen sind bis 0° unterkühlbar.

Beispiel 18

Eine flüssigkristalline Phase bestehend aus
38,3 % 2-p-Nonyloxyphenyl-5-nonylpyrimidin,
2,0 % 2-p-Hexyloxyphenyl-5-nonylpyrimidin,
36,1 % 2-p-Decyloxyphenyl-5-heptylpyrimidin,
5,9 % 2-p-Nonyloxyphenyl-5-heptylpyrimidin,
5,9 % 2-p-Octyloxyphenyl-5-heptylpyrimidin,
5,9 % 2-p-Heptyloxyphenyl-5-heptylpyrimidin und
5,9 % 2-p-Hexyloxyphenyl-5-heptylpyrimidin
ist bis unter 0° unterkühlbar und hat $S_C/S_A$ 53,5°, $S_A$/N 67,5° und N/I 70°.

Beispiel 19

Die flüssigkristalline Phase aus Beispiel 18 wird mit verschiedenen Mengen der chiralen Verbindung R-4-(5-hexylpyrimidyl-2)-phenyl-2-chlorpropionat dotiert. Die Phasenübergangstemperaturen sowie die Werte der spontanen Polarisation bei 20° der somit erhaltenen Mischungen sind in der folgenden Tabelle angegeben:

| Zugabe von X % des chiralen Dotierstoffes X | $S_C$* | $S_A$* | Ch | I | P |
|---|---|---|---|---|---|
| 3 | 48,1 | 65 | 68,7 | | 1,08 |
| 6 | 41,0 | 64,1 | 68,1 | | 2,14 |
| 10 | 34,5 | 63 | 68 | | 3,12 |

Die mit 10 % des chiralen Materials dotierte Mischung zeigt einen Tiltwinkel von 14,3° bei einer Pitchhöhe von 16,2 µm bei einer Temperatur von jeweils 20°.

Beispiel 20

Die flüssigkristalline Phase aus Beispiel 18 wird mit verschiedenen Mengen der chiralen Verbindung R-4-(5-Nonylpyrimidyl-2)-phenyl-2-chlorpropionat dotiert. Die Phasenübergangstemperaturen sowie die Werte der spontanen Polarisation der somit erhaltenen Mischungen sind in der folgenden Tabelle angegeben:

| Zugabe von X % des chiralen Dotierstoffes X | $S_C$* | $S_A$* | Ch | I P |
|---|---|---|---|---|
| 3 | 41 | 68 | 72 | 0,50 |
| 6 | 34,5 | 68,5 | 71 | 0,71 |
| 10 | 26,5 | 69 | 69,5 | 0,87 |

Beispiel 21

Die flüssigkristalline Phase aus Beispiel 18 wird mit verschiedenen Mengen der chiralen Verbindung R-4'-Nonyloxybiphenyl-4-yl-2-chlorpropionat dotiert. Die Phasenübergangstemperaturen sowie die Werte der spontanen Polarisation der somit erhaltenen Mischungen sind in der folgenden Tabelle angegeben. Die Werte für P gelten jeweils für 10° unterhalb des $S_C$*/$S_A$*-Übergangs.

| Zugabe von X % des chiralen Dotierstoffes X | $S_C$* | $S_A$* | Ch | I P |
|---|---|---|---|---|
| 0,5 | 50,1 | 64,2 | 69,3 | 0,1 |
| 2 | 45,7 | 64 | 69 | 0,2 |
| 3 | 42,3 | 63,7 | 68,6 | 0,3 |
| 4 | 40,8 | 63,1 | 68,4 | 0,5 |
| 5 | 37,1 | 62,7 | 68,1 | 0,6 |
| 10 | 24,5 | 61 | 67,9 | 0,8 |

Beispiel 22

Eine flüssigkristalline Phase bestehend aus
42 % 2-p-Decanoyloxyphenyl-5-octylpyrimidin,
23 % 2-p-Heptanoyloxyphenyl-5-octylpyrimidin,
18 % 2-p-Octanoyloxyphenyl-5-octylpyrimidin,
10 % 2-p-Hexanoyloxyphenyl-5-octylpyrimidin und
7 % 2-p-Nonanoyloxyphenyl-5-octylpyrimidin,
ist bis ca. 0° unterkühlbar und hat $S_C/S_A$ 52°, $S_A/N$ 54 ° und N/I 56°.

Beispiel 23

Eine flüssigkristalline Phase bestehend aus
42 % 2-p-Decanoyloxyphenyl-5-octylpyrimidin,
23 % 2-p-Heptanoyloxyphenyl-5-octylpyrimidin,
18 % 2-p-Octanoyloxyphenyl-5-octylpyrimidin,
10 % 2-p-Hexanoyloxyphenyl-5-octylpyrimidin und
7 % 2-p-Nonanoyloxyphenyl-5-octylpyrimidin,
7 % 2-p-Octyloxycarbonylphenyl-5-nonylpyrimidin ist bis ca. 0° unterkühlbar und hat $S_C/S_A$ 46,5 °, $S_A/N$ 53° und N/I 54,5°.

Beispiel 24

Eine flüssigkristalline Phase bestehend aus
34,5 % 2-p-Nonyloxyphenyl-5-nonylpyrimidin,
32,5 % 2-p-Decyloxyphenyl-5-heptylpyrimidin,
2,0 % 2-p-Hexyloxyphenyl-5-nonylpyrimidin,
5,0 % 2-p-Nonyloxyphenyl-5-heptylpyrimidin,
5,0 % 2-p-Octyloxyphenyl-5-heptylpyrimidin,
10,0 % 2-p-Hexyloxyphenyl-5-heptylpyrimidin und
11,0 % 2-p-Octylphenyl-5-p-pentyloxyphenylpyrimidin
ist bis ca. 10° unterkühlbar und hat $S_C/S_A$ 55°, $S_A/N$ 68,5° und N/I 75°.

Beispiel 25

Eine flüssigkristalline Phase bestehend aus
40 % 2-p-Decanoyloxyphenyl-5-octylpyrimidin,
20 % 2-p-Heptanoyloxyphenyl-5-octylpyrimidin,
20 % 2-p-Octanoyloxyphenyl-5-octylpyrimidin,
8 % 2-p-Hexanoyloxyphenyl-5-octylpyrimidin,
8 % 2-p-Octylphenyl-5-p-pentyloxyphenylpyrimidin und
4 % 2-p-Hexylphenyl-5-p-hexyloxyphenylpyrimidin
hat $S_C/S_A$ 57,5°, $S_A/N$ 67° und N/I 74°.

Beispiel 26

Eine flüssigkristalline Phase bestehend aus
23 % 2-p-Nonyloxyphenyl-5-nonylpyrimidin,
18 % 2-p-Decyloxyphenyl-5-heptylpyrimidin,
15 % 2-p-Nonyloxyphenyl-5-heptylpyrimidin,
8 % 2-p-Nonanoyloxyphenyl-5-nonylpyrimidin,
8 % 2-p-Undecanoyloxyphenyl-5-nonylpyrimidin,
10 % 2-p-Octylphenyl-5-p-octyloxyphenylpyrimidin,
7 % 2-p-Nonylphenyl-5-p-pentyloxyphenylpyrimidin und
11 % 2-p-Pentylphenyl-5-p-hexyloxyphenylpyrimidin
hat $S_C/S_A$ 63°, $S_A/N$ 74° und N/I 81°.

Beispiel 27

Man stellt eine flüssigkristalline Phase her bestehend aus
42 % 2-p-Decanoyloxyphenyl-5-octylpyrimidin,
23 % 2-p-Heptanoyloxyphenyl-5-octylpyrimidin,
18 % 2-p-Octanoyloxyphenyl-5-octylpyrimidin,
10 % 2-p-Hexanoyloxyphenyl-5-octylpyrimidin und
7 % 2-p-2-Oxadodecylphenyl-5-octylpyrimidin .

Beispiel 28

Man stellt eine flüssigkristalline Phase her bestehend aus
25 % 2-p-Decyloxyphenyl-5-heptylpyrimidin,
25 % 2-p-Nonyloxyphenyl-5-nonylpyrimidin,
10 % 2-p-Hexyloxyphenyl-5-nonylpyrimidin,
10 % 2-p-Nonyloxyphenyl-5-heptylpyrimidin,
11 % 2-p-Octyloxyphenyl-5-heptylpyrimidin,
11 % 2-p-Heptyloxyphenyl-5-heptylpyrimidin und
8 % 2-p-Decyloxyphenyl-5-(trans-4-octylcyclohexyl)-pyrimidin.

Beispiel 29

Man stellt eine flüssigkristalline Phase her bestehend aus
20 % 2-p-Nonyloxyphenyl-5-nonylpyrimidin,
10 % 2-p-Nonyloxyphenyl-5-heptylpyrimidin,
18 % 2-p-Decyloxyphenyl-5-octylpyrimidin,
15 % 2-p-Nonyloxyphenyl-5-octylpyrimidin,
15 % 2-p-Octyloxyphenyl-5-octylpyrimidin,
12 % 2-p-Heptyloxyphenyl-5-octylpyrimidin und
10 % 2-p-(1,4-Dioxaundecyl)-phenyl-5-nonylpyrimidin.

Alle als Beispiele aufgeführte achirale Basismischungen können mit einem geeigneten chiralen Dotierstoff versetzt werden und als ferroelektrische Materialien eingesetzt werden.

Beispiel 30

Zu einer Lösung von 0,01m (2,7 g) 3-(4-Heptyloxyphenyl)-pyridin (welches durch Kopplung von 4-Heptyloxyphenylmagnesiumbromid und 3-Brompyridin erhältlich ist) in 30 ml trockenem Toluol tropft man unter Stickstoff bei +15 °C eine Lösung von 0,01m n-Butyllithium (15 %ig in n-Pentan). Danach wird die Reaktionsmischung noch 4 h am Rückfluß gekocht und nach dem Abkühlen vorsichtig mit 10 ml Wasser hydrolysiert. Die organische Phase wird mit Wasser und gesättigter NaCl-Lösung gewaschen, mit Magnesiumsulfat getrocknet und eingedampft. Der ölige Rückstand wird chromatographisch über eine Kieselgelsäule mit Diisopropylether als Laufmittel gereinigt. Man erhält 2-Butyl-5-p-heptyloxyphenylpyridin, K. 80°.

Beispiel 31

Zu einer Lösung von 0,01m 3-Pentylpyridin, welches durch Kopplung von Pentylmagnesiumbromid und 3-Brom-pyridin erhältlich ist, in 30 ml Toluol tropft man bei -20 °C unter Stickstoff eine Lösung von 0,01m 4-Heptyloxyphenyllithium, dargestellt aus 4-Bromheptyloxybenzol und Lithium, in 30 ml Toluol. Die Reaktionsmischung wird 4 h zum Sieden erhitzt und nach dem Abkühlen vorsichtig mit 10 ml Wasser hydrolysiert. Die organische Phase wird mit Wasser und gesättigter NaCl-Lösung gewaschen, mit Magnesiumsulfat getrocknet und eingedampft. Der Rückstand wird über eine Kieselgelsäule mit Diisopropylether als Laufmittel chromatrographisch gereinigt. Man erhält 2-p-Heptyloxyphenyl-5-pentylpyrimidin.

Analog werden die homologen 2-p-Alkoxyphenyl-5-alkylpyridine hergestellt.

Beispiel 32

Eine flüssigkristalline Phase bestehend aus
3 % 2-p-Hexyloxyphenyl-5-heptylpyrimidin,

6 % 2-p-Hexyloxyphenyl-5-nonylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
20 % 2-p-Nonyloxyphenyl-5-nonylpyrimidin,
10 % 2-p-(trans-4-Propylcyclohexyl)-phenyl-5-pentylpyridin,
25 % r-1-Cyan-cis-4-(4'-octyloxybiphenyl-4-yl)-1-butylcyclohexan,
13 % r-1-Cyan-cis-4-(4'-heptylbiphenyl-4-yl)-1-hexylcyclohexan,
5 % r-1-Cyan-cis-4-(trans-4-pentylcyclohexyl)-1-(trans-4-pentylcyclohexyl)-cyclohexan und
9 % optisch aktives R-4-(5-Hexylpyrimidin-2-yl)-phenyl-2-chlorpropionat
zeigt K -10° Sc* 68° $S_A$* 73° Ch 99° I und eine spontane Polarisation von 8,1 nC/cm$^2$.

Beispiel 33

Eine flüssigkristalline Phase bestehend aus
3 % 2-p-Hexyloxyphenyl-5-heptylpyrimidin,
3 % 2-p-Hexyloxyphenyl-5-nonylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
22 % 2-p-Nonyloxyphenyl-5-nonylpyrimidin,
21 % r-1-Cyan-cis-4-(4'-octyloxybiphenyl-4-yl)-1-octylcyclohexan,
10 % r-1-Cyan-cis-4-(4'-heptylbiphenyl-4-yl)-1-hexylcyclohexan,
5 % 2-p-Pentyloxyphenyl-5-hexylpyridin,
5 % 2-p-Heptanoylphenyl-5-hexylpyridin,
10 % optisch aktives 1-(4'-Pentylbiphenyl-4-yl)-2-(1-cyan-3-methylcyclohexyl)-ethan und
12 % optisch aktives r-1-Cyan-cis-4-(4'-heptylbiphenyl-4-yl)-1-(2-methylbutyl)-cyclohexan
zeigt K -15° Sc* 58° $S_A$* 64° Ch 82° I und eine spontane Polarisation von 20 nC/cm$^2$.

Beispiel 34

Eine flüssigkristalline Phase bestehend aus
3 % 2-p-Hexyloxyphenyl-5-heptylpyrimidin,
3 % 2-p-Hexyloxyphenyl-5-nonylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
20 % 2-p-Nonyloxyphenyl-5-nonylpyrimidin,
3 % 2-p-Pentyloxyphenyl-5-hexylpyridin,
3 % 2-p-Hexyloxyphenyl-5-hexylpyridin,
3 % 2-p-Hexyloxyphenyl-5-octylpyridin,
30 % r-1-Cyan-cis-4-(4'-octyloxybiphenyl-4-yl)-1-octylcyclohexan,
16 % r-1-Cyan-cis-4-(4'-heptylbiphenyl-4-yl)-1-hexylcyclohexan und
10 % Ethyl-2-[p-(5-nonylpyrimidin-2-yl)-phenoxyl]-propanoat (optisch aktiv)
zeigt K -21° Sc* 61° $S_A$* 65° Ch 81° I und eine spontane Polarisation von 9 nC/cm$^2$.

Beispiel 35

Eine flüssigkristalline Phase bestehend aus
30 % 2-p-Nonyloxyphenyl-5-nonylpyrimidin,
3 % 2-p-Hexylmercaptophenyl-5-heptylpyrimidin,
3 % 2-p-Heptylmercaptophenyl-5-heptylpyrimidin,
3 % 2-p-Octylmercaptophenyl-5-heptylpyrimidin,
11 % r-1-Cyan-cis-4-(4'-pentylbiphenyl-4-yl)-1-octylcyclohexan,
10 % r-1-Cyan-cis-4-(4'-heptylbiphenyl-4-yl)-1-butylcyclohexan,
20 % r-1-Cyan-cis-4-(4'-nonyloxybiphenyl-4-yl)-1-octylcyclohexan und
10 % R-4-(5-Hexylpyrimidin-2-yl)-phenyl-2-chlorpropionat
zeigt K -10° Sc* 61° $S_A$* 66° Ch 85 I und eine spontane Polarisation von 12 nC/cm$^2$.

Beispiel 36

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
3 % 2-p-Hexyloxyphenyl-5-nonylpyrimidin,
20 % 2-p-Nonyloxyphenyl-5-nonylpyrimidin,
30 % r-1-Cyan-cis-4-(4'-octyloxybiphenyl-4-yl)-1-octylcyclohexan,
10 % r-1-Cyan-cis-4-(4'-heptylbiphenyl-4-yl)-1-hexylcyclohexan,
30 % r-1-Cyan-cis-4-(4'-octylbiphenyl-4-yl)-1-butylcyclohexan,
3 % p-Hexyloxyphenyl-5-heptylpicolinat,
3 % p-Hexyloxyphenyl-5-heptyloxypicolinat,
3 % p-Octyloxyphenyl-5-nonylpicolinat und
10 % chirales R-4-(5-Hexylpyrimidyl-2)-phenyl-2-chlorpropionat
zeigt K -12° Sc* 65° $S_A$* 69 Ch 89° I und eine spontane Polarisation von 12 nC/$cm^2$.

Beispiel 37

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
3 % 2-p-Hexyloxyphenyl-5-nonylpyrimidin,
20 % 2-p-Nonyloxyphenyl-5-nonylpyrimidin,
5 % 2-p-Pentyloxyphenyl-5-hexylpyridin,
5 % 2-p-Hexyloxyphenyl-5-heptylpyridin,
30 % r-1-Cyan-cis-4-(4'-octyloxybiphenyl-4-yl)-1-octylcyclohexan,
14 % optisch aktives r-1-Cyan-cis-4-(4'-heptyloxybiphenyl-4-yl)-1-(2-methylbutyl)-cyclohexan und
11 % optisch aktives 1-(4'-Pentylbiphenyl-4-yl)-2-(1-cyan-3-methylcyclohexyl)-ethan
zeigt K -25° Sc* 67 $S_A$* 74° Ch 88° I und eine spontane Polarisation von 27 nC/$cm^2$.

Beispiel 38

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
3 % 2-p-Hexyloxyphenyl-5-nonylpyrimidin,
20 % 2-p-Nonyloxyphenyl-5-nonylpyrimidin,
30 % r-1-Cyan-cis-4-(4'-octyloxybiphenyl-4-yl)-1-octylcyclohexan,
10 % r-1-Cyan-cis-4-(4'-heptylbiphenyl-4-yl)-1-hexylcyclohexan,
6 % r-1-Cyan-cis-4-(4'-octylbiphenyl-4-yl)-1-butylcyclohexan,
3 % p-Hexyloxybenzoesäure-(6-pentyloxypyridazin-3-ylester),
3 % p-Hexyloxybenzoesäure-(6-heptyloxyppyridazin-3-ylester),
3 % p-Heptyloxybenzoesäure-(6-heptyloxypyridazin-3-ylester) und
10 % chirales R-4-(5-Hexylpyrimidyl-2)-phenyl-2-chlorpropionat.

Beispiel 39

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,

3 % 2-p-Hexyloxyphenyl-5-nonylpyrimidin,
20 % 2-p-Nonyloxyphenyl-5-nonylpyrimidin,
30 % r-1-Cyan-cis-4-(4'-octyloxybiphenyl-4-yl)-1-octylcyclohexan,
10 % r-1-Cyan-cis-4-(4'-heptylbiphenyl-4-yl)-1-hexylcyclohexan,
6 % r-1-Cyan-cis-4-(4'-octylbiphenyl-4-yl)-1-butylcyclohexan,
3 % 6-(p-Heptyloxyphenyl)-3-hexylpyridazin,
3 % 6-(p-Heptyloxyphenyl)-3-heptylpyridazin,
3 % 6-(p-Nonyloxyphenyl)-3-heptylpyridazin und
10 % chirales R-4-(5-Hexylpyrimidyl-2)-phenyl-2-chlorpropionat.

Beispiel 40

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
3 % 2-p-Hexyloxyphenyl-5-nonylpyrimidin,
20 % 2-p-Nonyloxyphenyl-5-nonylpyrimidin,
30 % r-1-Cyan-cis-4-(4'-octyloxybiphenyl-4-yl)-1-octylcyclohexan,
10 % r-1-Cyan-cis-4-(4'-heptylbiphenyl-4-yl)-1-hexylcyclohexan,
6 % r-1-Cyan-cis-4-(4'-octylbiphenyl-4-yl)-1-butylcyclohexan,
3 % 1-(p-Heptyloxyphenyl)-2-(5-heptylpyridin-2-yl)-ethan,
3 % 1-(p-Nonyloxyphenyl)-2-(5-heptylpyridin-2-yl)-ethan,
3 % 1-(p-Nonyloxyphenyl)-2-(5-nonylpyridin-2-yl)-ethan, und
10 % chirales R-4-(5-Hexylpyrimidyl-2)-phenyl-2-chlorpropionat.

Beispiel 41

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
3 % 2-p-Hexyloxyphenyl-5-nonylpyrimidin,
20 % 2-p-Nonyloxyphenyl-5-nonylpyrimidin,
30 % r-1-Cyan-cis-4-(4'-octyloxybiphenyl-4-yl)-1-octylcyclohexan,
10 % r-1-Cyan-cis-4-(4'-heptylbiphenyl-4-yl)-1-hexylcyclohexan,
6 % r-1-Cyan-cis-4-(4'-octylbiphenyl-4-yl)-1-butylcyclohexan,
3 % 1-(trans-4-Heptylcyclohexyl)-2-(5-heptyloxypyridin-2-yl)-ethan,
3 % 1-(trans-4-Heptylcyclohexyl)-2-(5-octyloxypyridin-2-yl)-ethan,
3 % 1-(trans-4-Heptylcyclohexyl)-2-(5-nonyloxypyridin-2-yl)-ethan und
10 % chirales R-4-(5-Hexylpyrimidyl-2)-phenyl-2-chlorpropionat.

Beispiel 42

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
3 % 2-p-Hexyloxyphenyl-5-nonylpyrimidin,
20 % 2-p-Nonyloxyphenyl-5-nonylpyrimidin,
30 % r-1-Cyan-cis-4-(4'-octyloxybiphenyl-4-yl)-1-octylcyclohexan,
10 % r-1-Cyan-cis-4-(4'-heptylbiphenyl-4-yl)-1-hexylcyclohexan,
6 % r-1-Cyan-cis-4-(4'-octylbiphenyl-4-yl)-1-butylcyclohexan,
3 % 2-(4'-Heptyloxybiphenyl-4-yl)-5-heptylpyrimidin,
3 % 2-(4'-Octyloxybiphenyl-4-yl)-5-heptylpyrimidin,

3 % 2-(4'-Nonyloxybiphenyl-4-yl)-5-nonylpyrimidin und
10 % chirales R-4-(5-Hexylpyrimidyl-2)-phenyl-2-chlorpropionat.

Beispiel 43

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
3 % 2-p-Hexyloxyphenyl-5-nonylpyrimidin,
20 % 2-p-Nonyloxyphenyl-5-nonylpyrimidin,
30 % r-1-Cyan-cis-4-(4'-octyloxybiphenyl-4-yl)-1-octylcyclohexan,
10 % r-1-Cyan-cis-4-(4'-heptylbiphenyl-4-yl)-1-hexylcyclohexan,
6 % r-1-Cyan-cis-4-(4'-octylbiphenyl-4-yl)-1-butylcyclohexan,
3 % 2-(4'-Heptyloxybiphenyl-4-yl)-5-heptylpyridin,
3 % 2-(4'-Octyloxybiphenyl-4-yl)-5-heptylpyridin,
3 % 2-(4'-Nonyloxybiphenyl-4-yl)-5-nonylpyridin und
10 % chirales R-4-(5-Hexylpyrimidyl-2)-phenyl-2-chlorpropionat.

Beispiel 44

Man stellt eine flüssigkristalline Phase her bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
3 % 2-p-Hexyloxyphenyl-5-nonylpyrimidin,
20 % 2-p-Nonyloxyphenyl-5-nonylpyrimidin,
30 % r-1-Cyan-cis-4-(4'-octyloxybiphenyl-4-yl)-1-octylcyclohexan,
10 % r-1-Cyan-cis-4-(4'-heptylbiphenyl-4-yl)-1-hexylcyclohexan,
6 % r-1-Cyan-cis-4-(4'-octylbiphenyl-4-yl)-1-butylcyclohexan,
3 % 2-p-Heptyloxyphenyl-5-heptyloxypyrazin,
3 % 2-p-Nonyloxyphenyl-5-heptyloxypyrazin,
3 % 2-p-Nonyloxyphenyl-5-nonyloxypyrazin und
10 % chirales R-4-(5-Hexylpyrimidyl-2)-phenyl-2-chlorpropionat.

Beispiel 45

Eine flüssigkristalline Phase bestehend aus folgenden racemischen Verbindungen:
10 % 2-p-(4-Methylhexyloxyphenyl)-5-heptylpyrimidin (F. 29°, K. 60°)
6 % 2-p-(6-Methyloctyloxyphenyl)-5-octylpyrimidin (F. 3°, K. 56°),
8 % 2-p-(5-Methylheptyloxyphenyl)-5-nonylpyrimidin (F. 10°, K. 59°),
4 % 2-p-(4-Methylhexyloxyphenyl)-5-decylpyrimidin (F. 3°, K. 58°),
8 % 2-p-(5-Methylheptyloxyphenyl)-5-undecylpyrimidin (F. 20°, K. 59°),
3 % 2-p-(4-Methylhexyloxyphenyl)-5-dodecylpyrimidin (F. 41°, K. 62°),
6 % 2-p-(5-Methylheptyloxyphenyl)-5-dodecylpyrimidin (F. 23°, K. 62°) und
30 % r-1-Cyan-cis-4-(4'-octyloxybiphenyl-4-yl)-1-octylcyclohexan,
15 % r-1-Cyan-cis-4-(4'-octylbiphenyl-4-yl)-1-butylcyclohexan
sowie 10 % optisch aktives R-4-(5-Hexylpyrimidyl-2)-phenyl-2-chlorpropionat zeigt K -18° $S_C$* 66° $S_A$* 70° Ch 82° I, eine spontane Polarisation von 8 $nC/cm^2$ und eine Schaltzeit von 350 µs für 2 µm bei 12 V und 20°.

Beispiel 46

Eine flüssigkristalline Phase bestehend aus folgenden racemischen Verbindungen:
10 % 2-p-(4-Methylhexyloxyphenyl)-5-heptylpyrimidin (F. 29°, K. 60°)
6 % 2-p-(6-Methyloctyloxyphenyl)-5-octylpyrimidin (F. 3°, K. 56°),

8 % 2-p-(5-Methylheptyloxyphenyl)-5-nonylpyrimidin (F. 10°, K. 59°),
4 % 2-p-(4-Methylhexyloxyphenyl)-5-decylpyrimidin (F. 3°, K. 58°),
8 % 2-p-(5-Methylheptyloxyphenyl)-5-undecylpyrimidin (F. 20°, K. 59°),
3 % 2-p-(4-Methylhexyloxyphenyl)-5-dodecylpyrimidin (F. 41°, K. 62°),
6 % 2-p-(5-Methylheptyloxyphenyl)-5-dodecylpyrimidin (F. 23°, K. 62°),
30 % r-1-Cyan-cis-4-(4'-octyloxybiphenyl-4-yl)-1-octylcyclohexan,
15 % r-1-Cyan-cis-4-(4'-octylbiphenyl-4-yl)-1-butylcyclohexan,
6 % optisch aktives 1-(4'-Pentylbiphenyl-4-yl)-2-(1-cyan-3-methylcyclohexyl)-ethan und
4 % optisch aktives p-[p-(5-nonylpyrimidin-2-yl)-phenoxymethyl]-benzoesäure-2-octylester
zeigt K -15° Sc* 60°$S_A$* 66° Ch 80° I, eine spontane Polarisation von 9 nC/$cm^2$ und eine Schaltzeit von 300 µs für 2 µm bei 12 V und 20°.

Beispiel 47

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-(5-Methylheptyloxyphenyl)-5-heptylpyrimidin (Racemat),
3 % 2-p-Nonyloxyphenyl-5-heptylpyrimidin,
7 % 2-p-Hexyloxyphenyl-5-nonylpyrimidin,
23 % 2-p-Nonyloxyphenyl-5-nonylpyrimidin,
32 % r-1-Cyan-cis-4-(4'-octyloxybiphenyl-4-yl)-1-octylcyclohexan,
13 % r-1-Cyan-cis-4-(4'-heptylbiphenyl-4-yl)-1-hexylcyclohexan,
3 % r-1-Cyan-cis-4-(trans-4-pentylcyclohexyl)-1-(trans-4-pentylcyclohexyl)-cyclohexan und
10 % optisch aktives Ethyl-2-[p-(-5-nonylpyrimidin-2-yl)-phenoxy]-propanoat
zeigt K < -30° Sc* 59° $S_A$*.

Beispiel 48

Eine flüssigkristalline Phase bestehend aus
3 % 2-p-Hexyloxyphenyl-5-heptylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
3 % 2-p-Hexyloxyphenyl-5-nonylpyrimidin,
20 % 2-p-Nonyloxyphenyl-5-nonylpyrimidin,
15 % r-1-Cyan-cis-4-(4'-octylbiphenyl-4-yl)-1-butylcyclohexan,
30 % r-1-Cyan-cis-4-(4'-octyloxybiphenyl-4-yl)-1-octylcyclohexan,
3 % p-Octylthiobenzoesäure-S-(6-nonyloxypyridazin-3-ylester),
7 % optisch aktives 1-(4'-Pentylbiphenyl-4-yl)-2-(1-cyan-3-methylcyclohexyl)-ethan und
10 % optisch aktives r-1-Cyan-cis-4-(4'-heptyloxybiphenyl-4-yl)-1-(2-methylbutyl)-cyclohexan
zeigt Sc* 63° $S_A$* 66° Ch 84° I und eine spontane Polarisation von 20 nC/$cm^2$.

Bei den in den Beispielen 33, 37, 46 und 48 eingesetzten Gemischen von optisch aktiven Materialien ist jeweils ein Zusatz bestrebt, eine rechtshändige Verdrillung zu erzeugen, während der andere Zusatz bestrebt ist, eine linkshändige Verdrillung zu erzeugen.

Beispiel 49

Eine flüssigkristallne Phase bestehend aus
3 % 2-p-Hexyloxyphenyl-5-octylpyrimidin,
3 % 2-p-Heptyloxyphenyl-5-heptylpyrimidin,
3 % 2-p-Octyloxyphenyl-5-heptylpyrimidin,
3 % 2-p-Nonyloxyphenyl-5-heptylpyrimidin,
3 % 2-p-Hexyloxyphenyl-5-nonylpyrimidin,
20 % 2-p-Nonyloxyphenyl-5-nonylpyrimidin,
3 % 3-p-Pentyloxyphenyl-6-hexyloxypyridazin,
3 % 3-p-Pentyloxyphenyl-6-octyloxypyridazin,
3 % 3-p-Hexyloxyphenyl-6-hexyloxypyridazin,

25 % r-1-Cyan-cis-4-(4'-octylbiphenyl-4-yl)-1-butylcyclohexan,
10 % r-1-Cyan-cis-4-(4'-heptylbiphenyl-4-yl)-1-hexylcyclohexan,
5 % r-1-Cyan-cis-4-(trans-4-pentylcyclohexyl)-1-(trans-4-pentylcyclohexyl)-cyclohexan,
5 % r-1-Cyan-cis-4-(4'-nonyloxybiphenyl-4-yl)-1-octylcyclohexan und
11% optisch aktives Ethyl-2-[p-(5-Nonylpyrimidin-2-yl)-phenoxy]-propanoat
zeigt Sc* 58° $S_A$* 64° Ch 79° I und eine spontane Polarisation von 8 nC/$cm^2$.

**Patentansprüche**
**Patentansprüche für folgende Vertragsstaaten : CH, DE, FR, GB, LI**

**1.** Chirale getiltete smektische flüssigkristalline Phase, dadurch gekennzeichnet, daß sie mindestens drei Verbindungen der Formel I enthält,

$R^1$-$A^1$-$A^2$-$R^2$ I

worin
$R^1$ und $R^2$ jeweils Alkyl mit 1-15 C-Atomen, worin auch eine oder zwei nicht benachbarte $CH_2$-Gruppen durch -O-, -S-, -CO-, -O-CO-, -CO-O-, -CO-S-, -S-CO-, -CHHalogen-, -CHCN- und/oder -CH=CH- ersetzt sein können,
$A^1$ 1,4-Phenylen, trans-1,4-Cyclohexylen oder eine Einfachbindung, und
$A^2$

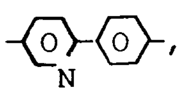, 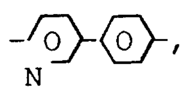, 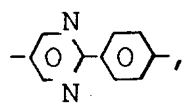, 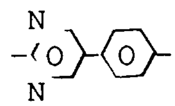

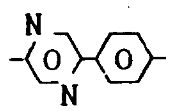, 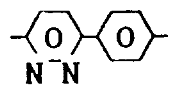, 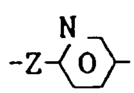, 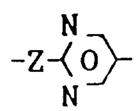,

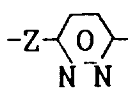 oder 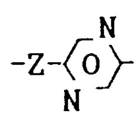

bedeutet,
wobei Z -O-CO-, -CO-O-, -S-CO-, -CO-S-, -$CH_2O$-, -$OCH_2$- oder -$CH_2CH_2$- bedeutet, enthält mit den Maßgaben, daß
a) $A^2$

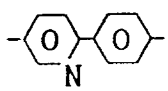, 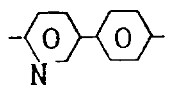, 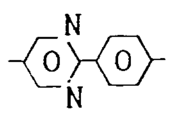, 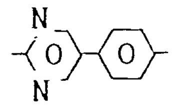

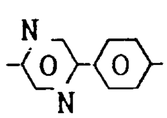 oder 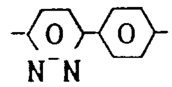

bedeutet, falls $A^1$ eine Einfachbindung ist, und
b) falls $A^1$ eine Einfachbindung ist und $A^2$

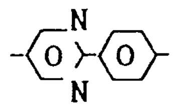

bedeutet,
Phasen enthaltend Verbindungen der Formel

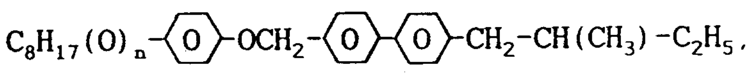

worin n 0 oder 1 ist, ausgenommen sind.

**2.** Chirale getiltete smektische flüssigkristalline Phase nach Anspruch 1, dadurch gekennzeichnet, daß sie keine chiralen Phenylbenzylether enthält.

**3.** Chirale getiltete smektische flüssigkristalline Phase nach Anspruch 1, dadurch gekennzeichnet, daß sie weniger als 40 % an chiralen Verbindungen enthält.

**4.** Chirale getiltete smektische flüssigkristalline Phase nach Anspruch 1, dadurch gekennzeichnet, daß sie ein oder zwei chirale Verbindungen enthält.

**5.** Chirale getiltete smektische flüssigkristalline Phase nach einem der Ansprüche 1 bis 4, dadurch gekennzeichnet, daß sie eine flüssigkristalline Komponente mit negativer dielektrischer Anisotropie enthält.

**6.** Phase nach Anspruch 5, dadurch gekennzeichnet, daß sie als Komponente mit negativer dielektrischer Anisotropie mindestens eine das Strukturelement A, B oder C

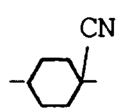
A

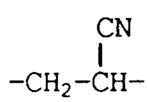
B

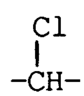
C

aufweisende Verbindung enthält.

**7.** Phase nach einem der Ansprüche 1 bis 6, dadurch gekennzeichnet, daß sie mindestens eine Verbindung der Formel II enthält,

$R^4$-$A^1$-COX-$A^2$-$R^5$ II

worin

| | |
|---|---|
| $R^4$ und $R^5$ | jeweils Alkyl mit 1-15 C-Atomen, worin auch eine oder zwei nicht benachbarte $CH_2$-Gruppen durch -O-, -CO-, -O-CO-, -CO-O-und/oder -CH=CH- ersetzt sein können, |
| X | O oder S, und |
| $A^1$ und $A^2$ | jeweils 1,4-Phenylen oder trans-1,4-Cyclohexylen bedeuten, eine der Gruppen $A^1$ und $A^2$ ggf. auch 4,4'-Biphenylyl oder trans,trans-4,4'-Bicyclohexyl, bedeuten. |

8. Phase nach einem der Ansprüche 1 bis 7, dadurch gekennzeichnet, daß
$A^1$ eine Einfachbindung ist, und
$A^2$

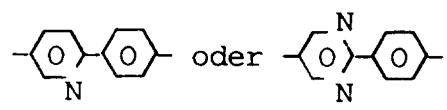

bedeutet.

9. Phase nach Anspruch 8, dadurch gekennzeichnet, daß
$R^1$ n-Alkyl mit 7 bis 10 C-Atomen, und
$R^2$ n-Alkoxy mit 6 bis 12 C-Atomen
bedeutet.

10. Phase nach Anspruch 9, dadurch gekennzeichnet, daß
$R^1$ n-Heptyl, n-Octyl oder n-Nonyl
bedeutet.

11. Phase nach einem der Ansprüche 8 bis 10, dadurch gekennzeichnet, daß die Summe der C-Atome der Gruppen $R^1$ und $R^2$ 15 oder mehr ist.

12. Phase nach Anspruch 11, dadurch gekennzeichnet, daß die Summe der C-Atome der Gruppen $R^1$ und $R^2$ 15 bis 20 ist.

13. Phase nach einem der Ansprüche 8 bis 12, dadurch gekennzeichnet, daß sie nur solche Verbindungen der Formel I nach Anspruch 1 enthält, worin $A^1$ eine Einfachbindung bedeutet.

14. Phase nach einem der Ansprüche 1 bis 13, dadurch gekennzeichnet, daß sie mindestens eine Verbindung der Formeln

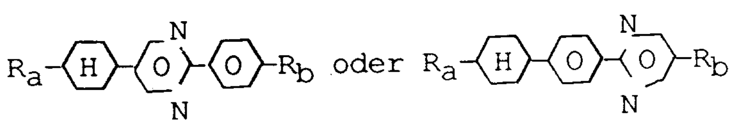

enthält, worin
$R_a$ Alkyl mit 3 bis 12 C-Atomen, und
$R_b$ Alkyl mit Alkoxy mit jeweils 5 bis 12 C-Atomen
bedeutet.

15. Phase nach einem der Ansprüche 1 bis 14, dadurch gekennzeichnet, daß sie mindestens eine Verbindung der Formel V enthält,

$R^1$-$Q^1$-A-$(Q^2)_q$-$R^2$ V

worin
$R^1$ und $R^2$ jeweils unabhängig voneinander eine geradkettige Alkylgruppe mit 1 bis 15 C-Atomen, worin auch eine oder mehrere nicht benachbarte $CH_2$-Gruppen durch -O-, -S-, -CO-, -$CHCH_3$-O-, -$CHCH_3$-, -CH-Halogen-, -CHCN-, -O-CO-, -O-COO-, -CO-O- und/oder -CH=CH-ersetzt sein können,
A

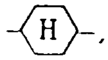, 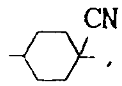, 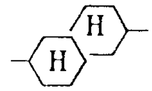,

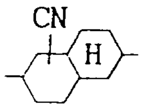, 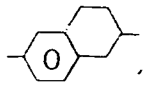, 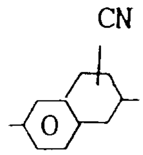,

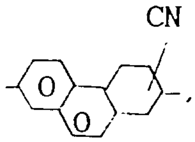, 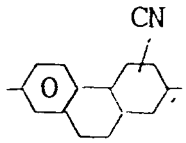

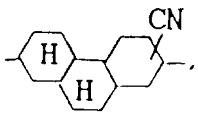, 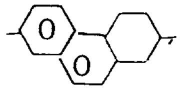

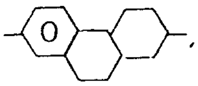, 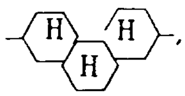,

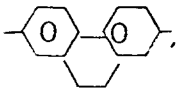, 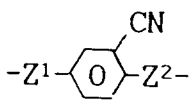 oder

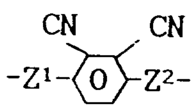

q 0 oder 1,

$Q^1$ und $Q^2$ jeweils unabhängig voneinander, $-(A°-Z°)_p-$,wobei

A° unsubstituiertes oder ein- oder mehrfach durch Halogenatome, $CH_3$- und/oder Nitril-Gruppen substituiertes 1,4-Cyclohexylen, worin auch eine oder zwei nicht benachbarte $CH_2$-Gruppen durch -O- und/oder -S- und/oder eine

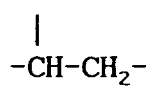

Gruppierung durch

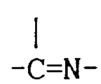

ersetzt sein können (Cy), oder unsubstituiertes oder ein- oder mehrfach durch Halogenatome, $CH_3$- und/oder Nitril-Gruppen substituiertes 1,4-Phenylen, worin auch eine oder mehrere CH-Gruppen durch N ersetzt sein können (Ph), bedeutet, einer der Reste A° auch 2,6-Naphthylen (Na) oder Tetrahydro-2,6-naphthylen (4H-Na), gegebenenfalls durch Halogen oder CN substituiert,

| | |
|---|---|
| $Z^{\circ}$, $Z^1$ und $Z^2$ | jeweils unabhängig voneinander -CO-O-, -O-CO-, $-CH_2O-$, $-OCH_2-$, $-CH_2CH_2-$, $-CHCNCH_2-$, $-CH_2-CHCN-$ oder eine Einfachbindung, und |
| p | 1, 2 oder 3, oder im Falle A = Tetra- oder Octahydrophenanthren auch O bedeutet, wobei im Falle A = |

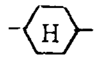, 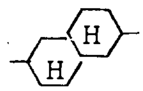, 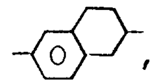,
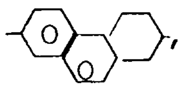, 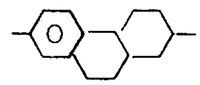,
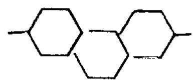 oder 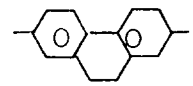,

mindestens eine Gruppe $Z^{\circ}$ $-CHCNCH_2-$ oder $-CH_2CHCN-$ bedeutet und/oder in mindestens einer der Gruppen $R^1$ und $R^2$ mindestens eine $CH_2$-Gruppe durch -CHCN- ersetzt ist.

**16.** Phase nach Anspruch 15, dadurch gekennzeichnet, daß achirale Basismischungen aus Verbindungen der Formel V mit chiralen Verbindungen der Formel I oder auch zusätzlich mit anderen chiralen Verbindungen dotiert sind.

**17.** Phase nach Anspruch 15, dadurch gekennzeichnet, daß sie mindestens eine Verbindung ausgewählt aus den Formeln VI bis V18 enthält:

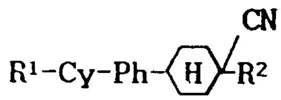

V1

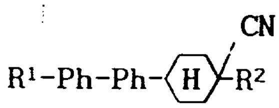

V2

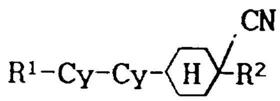

V3

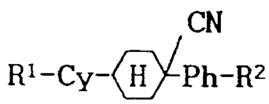

V4

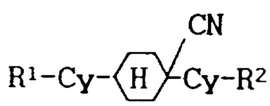

V5

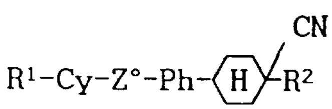

V6

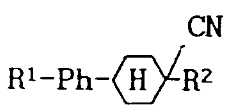

V7

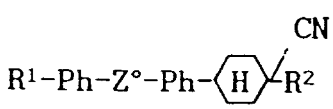

V8

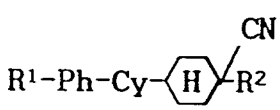

V9

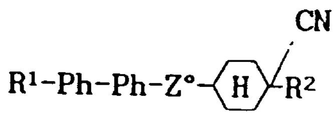

V10

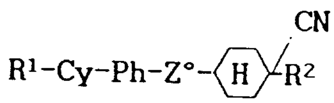

V11

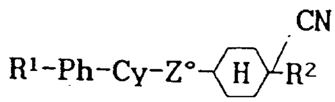

V12

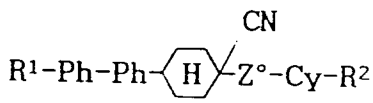

V13

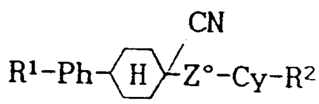

V14

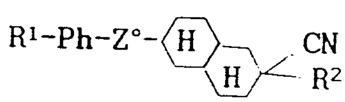

V15

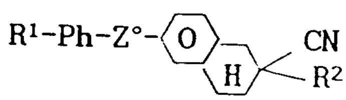

V16

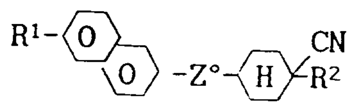

V17

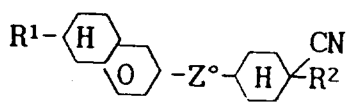

V18

worin $R^1$, $R^2$, Ph, Cy und Z° die angegebene Bedeutung besitzen.

**18.** Phase nach Anspruch 15, dadurch gekennzeichnet, daß sie mindestens eine Verbindung ausgewählt aus den Formeln V19 bis V22 enthält:

$R^1$-A°-Cy-$(CH_2)_r$-CHCN-$C_sH_{2s+1}$ V19

$R^1$-A°-A°-Cy-$(CH_2)_r$-CHCN-$C_sH_{2s+1}$ V20

$R^1$-A°-A°-CHCN-$CH_2$-Cy-$R^2$ V21

$R^1$-A°-A°-$CH_2$-CHCN-Cy-$R^2$ V22

worin r 0, 1, 2 oder 3 bedeutet und (r + s) 1 bis 14 ist.

Verbindungen der Formel I, die keine $S_c$-Phasen aufweisen, eignen sich ebenfalls als Komponenten erfindungsgemäßer smektischer Phasen.

**19.** Phase nach einem der Ansprüche 1 bis 18, dadurch gekennzeichnet, daß sie mindestens eine Verbindung der Formel IIIc enthält,

R'-$Q^3$-$Q^4$-R''' IIIc

worin

R geradkettiges Alkyl- oder Alkoxy mit jeweils 2 bis 10 C-Atomen,

$Q^3$ und $Q^4$ jeweils 1,4-Phenylen, 4,4'-Biphenylen, oder trans-1,4-Cyclohexylen, eine der Gruppen $Q^3$ und $Q^4$ auch 1,4-Phenylen, worin mindestens eine CH-Gruppe durch N ersetzt ist, und

R''' ein optisch aktiver Rest mit einem asymetrischen Hohlenstoffatom der Struktur

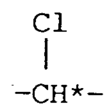

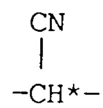

bedeuten.

**20.** Phase nach Anspruch 21, dadurch gekennzeichnet, daß sie eine Verbindung der Formel IIIc' enthält,

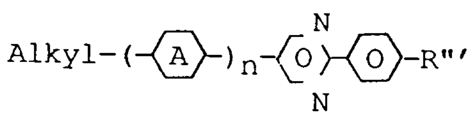 IIIc'

worin

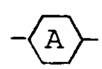

1,4-Phenylen oder trans-1,4-cyclohexylen bedeutet, und

n 0 oder 1 ist.

**21.** Elektrooptisches Anzeigeelement, dadurch gekennzeichnet, daß es als Dielektrikum eine Phase nach einem der Ansprüche 1 bis 20 enthält.

**Patentansprüche für folgende Vertragsstaaten : AT, BE, IT, NL, SE**

**1.** Chirale getiltete smektische flüssigkristalline Phase, dadurch gekennzeichnet, daß sie mindestens drei Verbindungen der Formel I enthält,

$R^1$-$A^1$-$A^2$-$R^2$ I

worin

$R^1$ und $R^2$ jeweils Alkyl mit 1-15 C-Atomen, worin auch eine oder zwei nicht benachbarte $CH_2$-Gruppen durch -O-, -S-, -CO-, -O-CO-, -CO-O-, -CO-S-, S-CO-, -CHHalogen-, -CHCN- und/oder -CH=CH- ersetzt sein können,

$A^1$ 1,4-Phenylen, trans-1,4-Cyclohexylen oder eine Einfachbindung, und

$A^2$

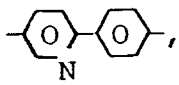, 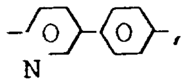, 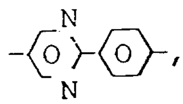, 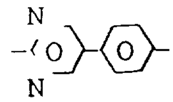

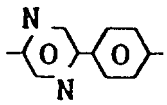, 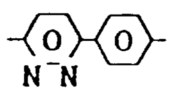, 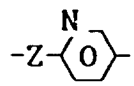, 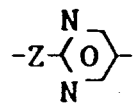,

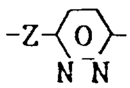 oder 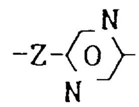

bedeutet,
wobei Z -O-CO-, -CO-O-, -S-CO-, -CO-S-, $-CH_2O-$, $-OCH_2-$ oder $-CH_2CH_2-$ bedeutet, enthält mit der Maßgaben, daß

$A^2$

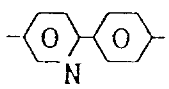, 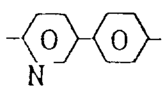, 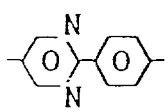, 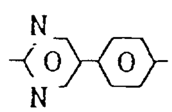

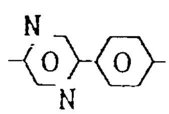 oder 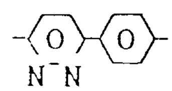

bedeutet, falls $A^1$ eine Einfachbindung ist,

2. Chirale getiltete smektische flüssigkristalline Phase nach Anspruch 1, dadurch gekennzeichnet, daß sie keine chiralen Phenylbenzylether enthält.

3. Chirale getiltete smektische flüssigkristalline Phase nach Anspruch 1, dadurch gekennzeichnet, daß sie weniger als 40 % an chiralen Verbindungen enthält.

4. Chirale getiltete smektische flüssigkristalline Phase nach Anspruch 1, dadurch gekennzeichnet, daß sie ein oder zwei chirale Verbindungen enthält.

5. Chirale getiltete smektische flüssigkristalline Phase nach einem der Ansprüche 1 bis 4, dadurch gekennzeichnet, daß sie eine flüssigkristalline Komponente mit negativer dielektrischer Anisotropie enthält.

6. Phase nach Anspruch 5, dadurch gekennzeichnet, daß sie als Komponente mit negativer dielektrischer Anisotropie mindestens eine das Strukturelement A, B oder C

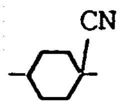 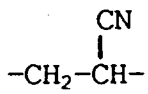 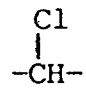

A B C

aufweisende Verbindung enthält.

**7.** Phase nach einem der Ansprüche 1 bis 6, dadurch gekennzeichnet, daß sie mindestens eine Verbindung der Formel II enthält,

$R^4$-$A^1$-COX-$A^2$-$R^5$ II

worin

| | |
|---|---|
| $R^4$ und $R^5$ | jeweils Alkyl mit 1-15 C-Atomen, worin auch eine oder zwei nicht benachbarte $CH_2$-Gruppen durch -O-, -CO-, -O-CO-, -CO-O-und/oder -CH = CH- ersetzt sein können, |
| X | O oder S, und |
| $A^1$ und $A^2$ | jeweils 1,4-Phenylen oder trans-1,4-Cyclohexylen bedeuten, eine der Gruppen $A^1$ und $A^2$ ggf. auch 4,4'-Biphenylyl oder trans,trans-4,4'-Bicyclohexyl, bedeuten. |

**8.** Phase nach einem der Ansprüche 1 bis 7, dadurch gekennzeichnet, daß
$A^1$ eine Einfachbindung ist, und
$A^2$

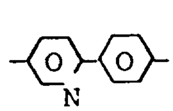 oder 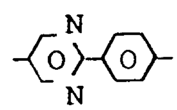

bedeutet.

**9.** Phase nach Anspruch 8, dadurch gekennzeichnet, daß
$R^1$ n-Alkyl mit 7 bis 10 C-Atomen, und
$R^2$ n-Alkoxy mit 6 bis 12 C-Atomen
bedeutet.

**10.** Phase nach Anspruch 9, dadurch gekennzeichnet, daß
$R^1$ n-Heptyl, n-Octyl oder n-Nonyl
bedeutet.

**11.** Phase nach einem der Ansprüche 8 bis 10, dadurch gekennzeichnet, daß die Summe der C-Atome der Gruppen $R^1$ und $R^2$ 15 oder mehr ist.

**12.** Phase nach Anspruch 11, dadurch gekennzeichnet, daß die Summe der C-Atome der Gruppen $R^1$ und $R^2$ 15 bis 20 ist.

**13.** Phase nach einem der Ansprüche 8 bis 12, dadurch gekennzeichnet, daß sie nur solche Verbindungen der Formel I nach Anspruch 1 enthält, worin $A^1$ eine Einfachbindung bedeutet.

**14.** Phase nach einem der Ansprüche 1 bis 13, dadurch gekennzeichnet, daß sie mindestens eine Verbindung der Formeln

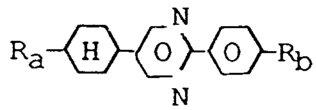 oder 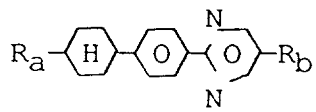

enthält, worin

$R_a$ Alkyl mit 3 bis 12 C-Atomen, und

$R_b$ Alkyl mit Alkoxy mit jeweils 5 bis 12 C-Atomen

bedeutet.

**15.** Phase nach einem der Ansprüche 1 bis 14, dadurch gekennzeichnet, daß sie mindestens eine Verbindung der Formel V enthält,

$R^1$-$Q^1$-A-$(Q^2)_q$-$R^2$ V

worin

$R^1$ und $R^2$ jeweils unabhängig voneinander eine geradkettige Alkylgruppe mit 1 bis 15 C-Atomen, worin auch eine oder mehrere nicht benachbarte $CH_2$-Gruppen durch -O-, -S-, -CO-, $-CHCH_3$-O-, $-CHCH_3$-, -CH-Halogen-, -CHCN-, -O-CO-, -O-COO-, -CO-O- und/oder -CH = CH-ersetzt sein können,

A

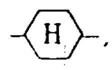, 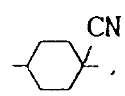, 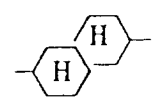,

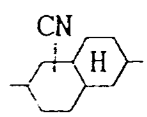, 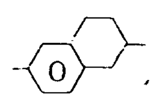, 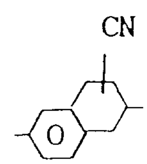,

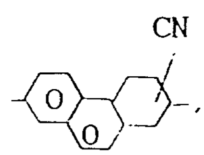, 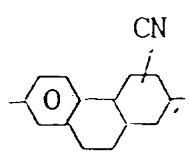

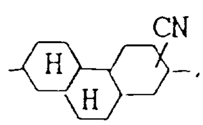, 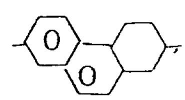

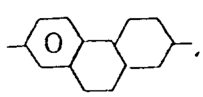, 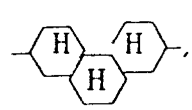,

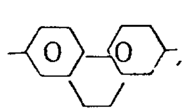, 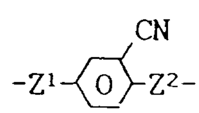 oder

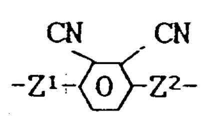

q — 0 oder 1,

$Q^1$ und $Q^2$ — jeweils unabhängig voneinander, -(A°-Z°)$_p$-,wobei

A° — unsubstituiertes oder ein- oder mehrfach durch Halogenatome, $CH_3$- und/oder Nitril-Gruppen substituiertes 1,4-Cyclohexylen, worin auch eine oder zwei nicht benachbarte $CH_2$-Gruppen
durch -O- und/oder -S- und/oder eine

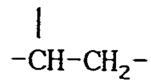

Gruppierung durch

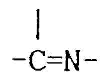

ersetzt sein können (Cy), oder unsubstituiertes oder ein- oder mehrfach durch Halogenatome, $CH_3$- und/oder Nitril-Gruppen substituiertes 1,4-Phenylen, worin auch eine oder mehrere CH-Gruppen durch N ersetzt sein können (Ph), bedeutet, einer der Reste A° auch 2,6-Naphthylen (Na) oder Tetrahydro-2,6-naphthylen (4H-Na), gegebenenfalls durch Halogen oder CN substituiert,

Z°, $Z^1$ und $Z^2$ — jeweils unabhängig voneinander -CO-O-, -O-CO-, -$CH_2$O-, -O$CH_2$-, -$CH_2CH_2$-, -CHCN$CH_2$-, -$CH_2$-CHCN- oder eine Einfachbindung, und

p — 1, 2 oder 3, oder im Falle A = Tetra- oder Octahydrophenanthren auch O bedeutet, wobei im Falle A =

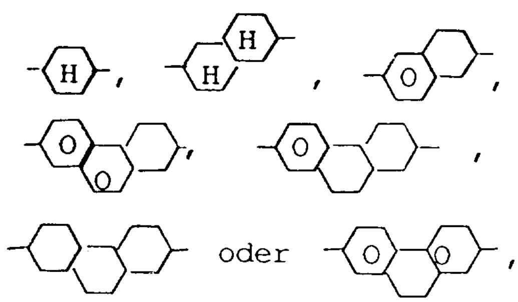

mindestens eine Gruppe Z° -CHCN$CH_2$- oder -$CH_2$CHCN- bedeutet und/oder in mindestens eine der Gruppen $R^1$ und $R^2$ mindestens eine $CH_2$-Gruppe durch -CHCN- ersetzt ist.

**16.** Phase nach Anspruch 15, dadurch gekennzeichnet, daß achirale Basismischungen aus Verbindungen der Formel V mit chiralen Verbindungen der Formel I oder auch zusätzlich mit anderen chiralen Verbindungen dotiert sind.

**17.** Phase nach Anspruch 15, dadurch gekennzeichnet, daß sie mindestens eine Verbindung ausgewählt aus den Formeln V1 bis V18 enthält:

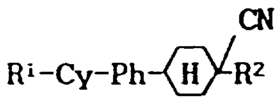

V1

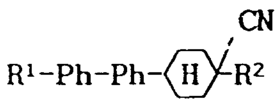

V2

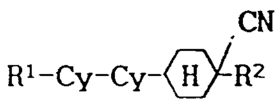

V3

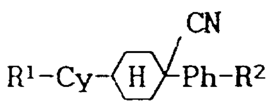

V4

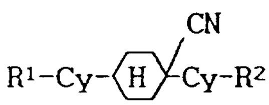

V5

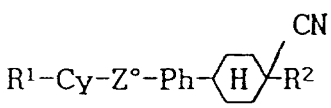

V6

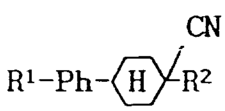

V7

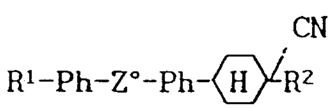

V8

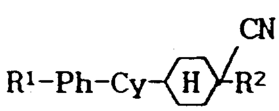

V9

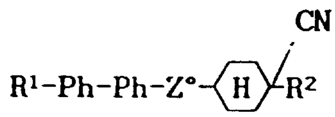

V10

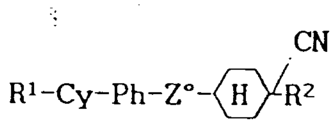

V11

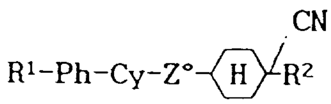

V12

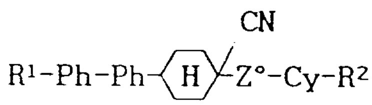

V13

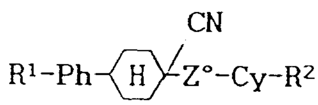

V14

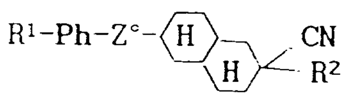

V15

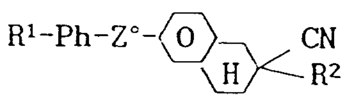

V16

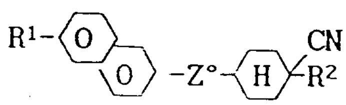

V17

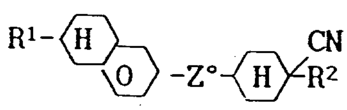

V18

worin $R^1$, $R^2$, Ph, Cy und Z° die angegebene Bedeutung besitzen.

**18.** Phase nach Anspruch 15, dadurch gekennzeichnet, daß sie mindestens eine Verbindung ausgewählt aus den Formeln V19 bis V22 enthält:

$R^1$-A°-Cy-$(CH_2)_r$-CHCN-$C_sH_{2s+1}$ V19

$R^1$-A°-A°-Cy$(CH_2)_r$-CHCN-$C_sH_{2s+1}$ V20

$R^1$-A°-A°-CHCN-$CH_2$-Cy-$R^2$ V21

$R^1$-A°-A°-$CH_2$-CHCN-Cy-$R^2$ V22

worin r 0, 1, 2 oder 3 bedeutet und (r + s) 1 bis 14 ist.

Verbindungen der Formel I, die keine $S_c$-Phasen aufweisen, eignen sich ebenfalls als Komponenten erfindungsgemäßer smektischer Phasen.

**19.** Phase nach einem der Ansprüche 1 bis 18, dadurch gekennzeichnet, daß sie mindestens eine Verbindung der Formel IIIc enthält,

R'-$Q^3$-$Q^4$-R'''   IIIc

worin

| | |
|---|---|
| R | geradkettiges Alkyl- oder Alkoxy mit jeweils 2 bis 10 C-Atomen, |
| $Q^3$ und $Q^4$ | jeweils 1,4-Phenylen, 4,4'-Biphenylen, oder trans-1,4-Cyclohexylen, eine der Gruppen $Q^3$ und $Q^4$ auch 1,4-Phenylen, worin mindestens eine CH-Gruppe durch N ersetzt ist, und |
| R''' | ein optisch aktiver Rest mit einem asymetrischen Kohlenstoffatom der Struktur |

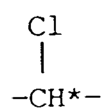 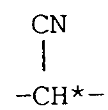

bedeuten.

**20.** Phase nach Anspruch 21, dadurch gekennzeichnet, daß sie eine Verbindung der Formel IIIc' enthält,

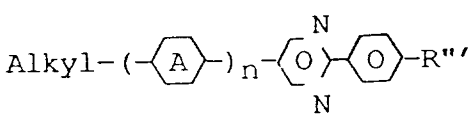

IIIc'

worin

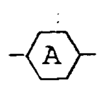

1,4-Phenylen oder trans-1,4-cyclohexylen bedeutet, und

n   0 oder 1 ist.

**21.** Elektrooptisches Anzeigeelement, dadurch gekennzeichnet, daß es als Dielektrikum eine Phase nach einem der Ansprüche 1 bis 20 enthält.

**Claims**
**Claims for the following Contracting States : CH, DE, FR, GB, LI**

**1.** Chiral tilted smectic liquid-crystalline phase, characterized in that it contains contains [sic] at least three compounds of the formula I,

$R^1$-$A^1$-$A^2$-$R^2$   I

in which
$R^1$ and $R^2$ are each alkyl having 1-15 C atoms, in which one or two non-adjacent $CH_2$ groups can also be replaced by -O-, -S-, -CO-, -O-CO-, -CO-O-, -CO-S-, -S-CO-, -CHhalogen-, -CHCN- and/or -CH = CH-
,

$A^1$   is 1,4-phenylene, trans-1,4-cyclohexylene or a single bond, and
$A^2$   is

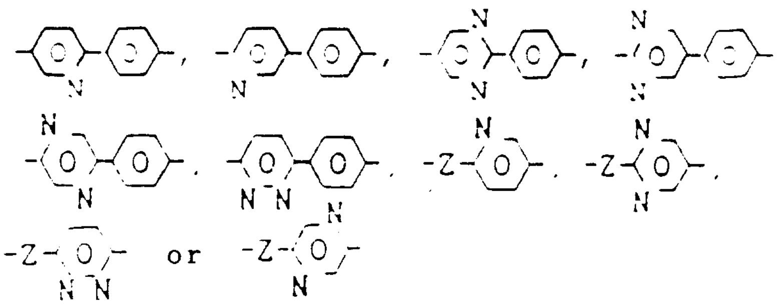

or

Z being -O-CO-, -CO-O-, -S-CO-, -CO-S-, $-CH_2O-$, $-OCH_2-$ or $-CH_2CH_2-$, with the provisos that

a) $A^2$ is

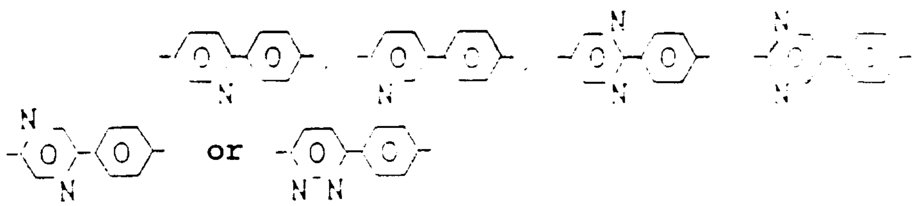

or

if $A^1$ is a single bond, and

b) if $A^1$ is a single bond and $A^2$ is

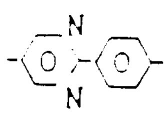

phases containing compounds of the formula

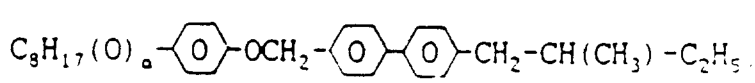

where n is 0 or 1 are excepted.

**2.** Chiral tilted smectic liquid-crystalline phase according to Claim 1, characterised in that it does not contain any chiral phenyl benzyl ethers.

**3.** Chiral tilted smectic liquid-crystalline phase according to Claim 1, characterised in that it contains less than 40% of chiral compounds.

**4.** Chiral tilted smectic liquid-crystalline phase according to Claim 1, characterised in that it contains one or two chiral compounds.

**5.** Chiral tilted smectic liquid-crystalline phase according to one of Claims 1 to 4, characterised in that it contains a liquid-crystalline component having negative dielectric anisotropy.

**6.** Phase according to Claim 5, characterised in that it contains at least one compound containing the structural element A, B or C

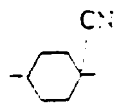 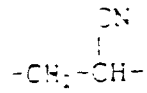 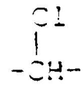

A B C

as the component having negative dielectric anisotropy.

**7.** Phase according to one of Claims 1 to 6, characterised in that it contains at least one compound of the formula II,

$R^4$-$A^1$-COX-$A^2$-$R^5$ II

in which

$R^4$ and $R^5$ are each alkyl having 1-15 C atoms, in which one or two non-adjacent $CH_2$ groups can also be replaced by -O-, -CO-, -O-CO-, -CO-O- and/or - CH = CH-,

X is O or S, and

$A^1$ and $A^2$ are each 1,4-phenylene or trans-1,4-cyclohexylene, one of the groups $A^1$ and $A^2$ is also, if desired, 4,4'-biphenylyl or trans,trans-4,4'-bicyclohexyl.

**8.** Phase according to one of Claims 1 to 7, characterised in that

$A^1$ is a single bond and

$A^2$ is

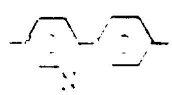 or 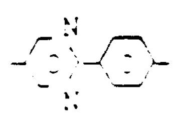 .

**9.** Phase according to Claim 8, characterised in that

$R^1$ is n-alkyl having 7 to 10 C atoms and

$R^2$ is n-alkoxy having 6 to 12 C atoms.

**10.** Phase according to Claim 9, characterised in that

$R^1$ is n-heptyl, n-octyl or n-nonyl.

**11.** Phase according to one of Claims 8 to 10, characterised in that the sum of C atoms of groups $R^1$ and $R^2$ is 15 or more.

**12.** Phase according to Claim 11, characterised in that the sum of C atoms of groups $R^1$ and $R^2$ is 15 to 20.

**13.** Phase according to one of Claims 8 to 12, characterised in that it contains only those compounds of the formula I according to Claim 1 in which $A^1$ is a single bond.

**14.** Phase according to one of Claims 1 to 13, characterised in that it contains at least one compound of the formulae

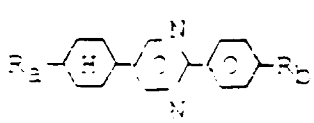 or 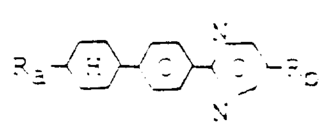

in which

$R_a$ is alkyl having 3 to 12 C atoms and

$R_b$ is alkyl with [sic] alkoxy each having 5 to 12 C atoms.

**15.** Phase according to one of Claims 1 to 14, characterised in that it contains at least one compound of the formula V,

$R^1$-$Q^1$-A-$(Q^2)_q$-$R^2$ V

in which

$R^1$ and $R^2$ are each, independently of one another, a straight-chain alkyl group having 1 to 15 C atoms, in which one or more non-adjacent $CH_2$ groups can also be replaced by -O-, -S-, -CO-, -$CHCH_3$-O-, -$CHCH_3$-, -CH-halogen-, -CHCN-, -O-CO-, -O-COO-, -CO-O- and/or -CH=CH-,

A is

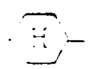
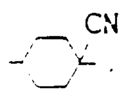
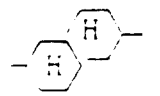
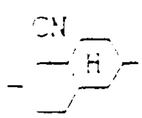
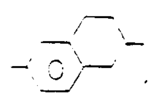
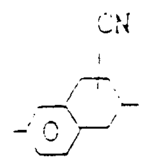
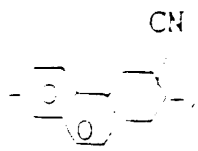
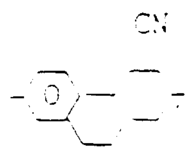
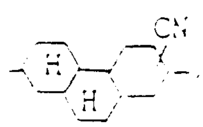
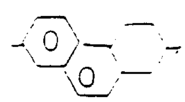
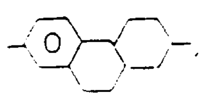
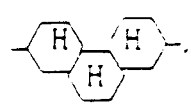
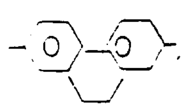
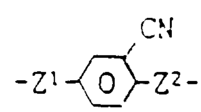

or

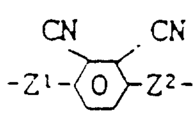

q is 0 or 1,

$Q^1$ and $Q^2$ are each, independently of one another, -(A°-Z°)$_p$-, in which

A° is 1,4-cyclohexylene which is unsubstituted or mono- or polysubstituted by halogen atoms, $CH_3$ and/or nitrile groups and in which one or two non-adjacent $CH_2$ groups can also be replaced by -O- and/or -S- and/or a

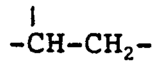

grouping by

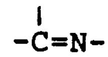

(Cy), or is 1,4-phenylene which is unsubstituted or mono- or polysubstituted by halogen atoms, $CH_3$ and/or nitrile groups and in which one or more CH groups can also be replaced by N (Ph), one of the radicals A° also [lacuna] 2,6-naphthylene (Na) or tetrahydro-2,6-naphthylene (4H-Na) each of which is unsubstituted or substituted by halogen or CN,

Z°, $Z^1$ and $Z^2$ are each, independently of one another, -CO-O-, -O-CO-, $-CH_2O-$, $-OCH_2-$, $-CH_2CH_2-$, $-CHCNCH_2-$, - $CH_2$-CHCN- or a single bond, and

p is 1, 2 or 3, or, in the case where A is tetra- or octahydrophenanthrene, is also 0, at least one group Z° in the case where A is

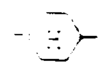, 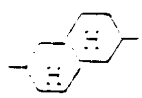, 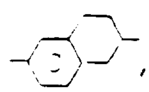,

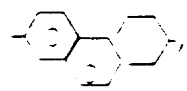, 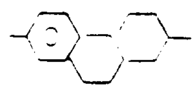,

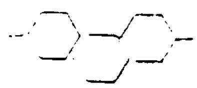 or 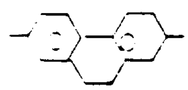,

being $-CHCNCH_2-$ or $-CH_2CHCN-$ and/or at least one $CH_2$ group being replaced in at least one of the groups $R^1$ and $R^2$ by -CHCN-.

**16.** Phase according to Claim 15, characterised in that achiral base mixtures composed of compounds of the formula V have been doped with chiral compounds of the formula I or else additionally with other chiral compounds.

**17.** Phase according to Claim 15, characterised in that it contains at least one compound selected from formulae V1 to V18:

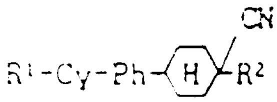

V1

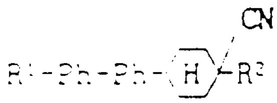

V2

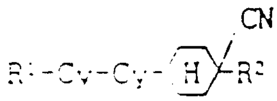

V3

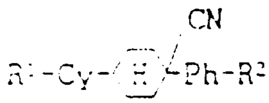

V4

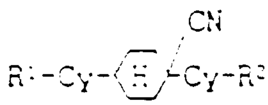

V5

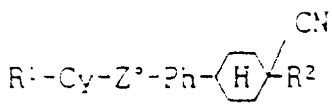

V6

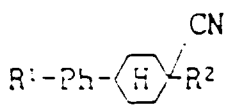

V7

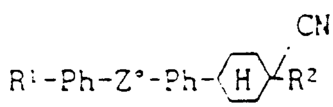

V8

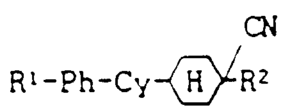

V9

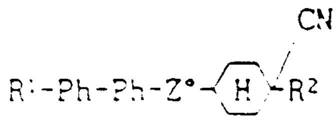
V10

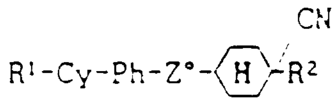
V11

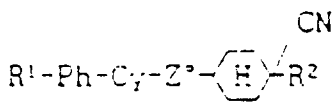
V12

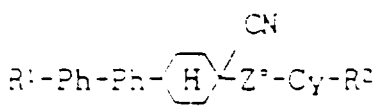
V13

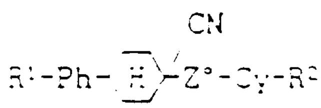
V14

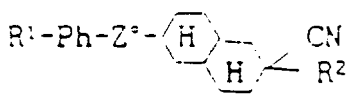
V15

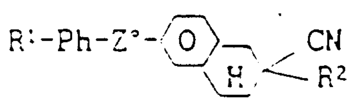
V16

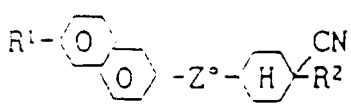
V17

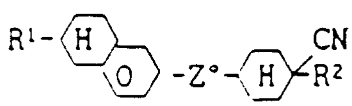
V18

in which $R^1$, $R^2$, Ph, Cy and Z° have the meaning given.

**18.** Phase according to Claim 15, characterised in that it contains at least one compound selected from formulae V19 to V22:

$R^1$-A°-Cy-$(CH_2)_r$-CHCN-$C_sH_{2s+1}$ V19

$R^1$-A°-A°-Cy-$(CH_2)_r$-CHCN-$C_sH_{2s+1}$ V20

$R^1$-A°-A°-CHCN-$CH_2$-Cy-$R^2$ V21

$R^1$-A°-A°$CH_2$-CHCN-Cy-$R^2$ V22

in which r is 0, 1, 2 or 3 and (r + s) is 1 to 14.
Compounds of the formula I which do not exhibit $S_c$ phases are also suitable as components of smectic phases according to the invention.

**19.** Phase according to one of Claims 1 to 18, characterized in that it contains at least one compound of the formula IIIc

R'-$Q^3$-$Q^4$-R'''  IIIc

in which

| | |
|---|---|
| R | is straight-chain alkyl or alkoxy each having 2 to 10 C atoms, |
| $Q^3$ and $Q^4$ | are each 1,4-phenylene, 4,4'-biphenylene, or trans-1,4-cyclohexylene, one of the groups $Q^3$ and $Q^4$ is also 1,4-phenylene in which at least one CH group is replaced by N, and |
| R''' | is an optically active radical having an asymmetric carbon atom of the structure |

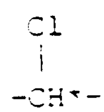
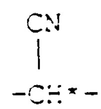
.

**20.** Phase according to Claim 21 [sic], characterised in that it contains a compound of the formula IIIc',

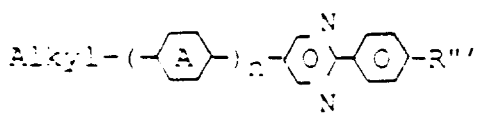
IIIc'

in which

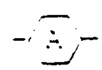

is 1,4-phenylene or trans-1,4-cyclohexylene, and n is 0 or 1.

**21.** Electro-optical display element, characterised in that it contains a phase according to one of Claims 1 to 20 as the dielectric.

**Claims for the following Contracting States : AT, BE, IT, NL, SE**

**1.** Chiral tilted smectic liquid-crystalline phase, characterized in that it contains contains [sic] at least three compounds of the formula I,

$R^1$-$A^1$-$A^2$-$R^2$  I

in which
$R^1$ and $R^2$ are each alkyl having 1-15 C atoms, in which one or two non-adjacent $CH_2$ groups can also be replaced by -O-, -S-, -CO-, -O-CO-, -CO-O-, -CO-S-, -S-CO-, -CHhalogen-, -CHCN- and/or -CH = CH-,

| | |
|---|---|
| $A^1$ | is 1,4-phenylene, trans-1,4-cyclohexylene or a single bond, and |
| $A^2$ | is |

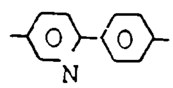, 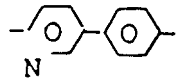, 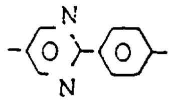, 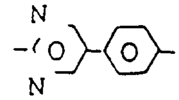
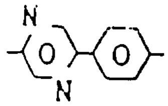, 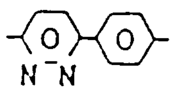, 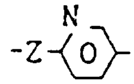, 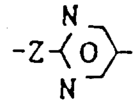,
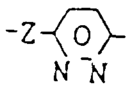 or 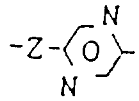

Z being -O-CO-, -CO-O-, -S-CO-, -CO-S-, $-CH_2O-$, $-OCH_2-$ or $-CH_2CH_2-$, with the provisos that

$A^2$ is

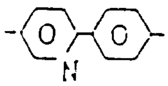, 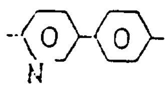, 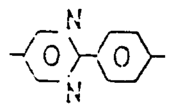, 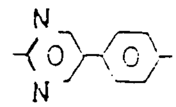
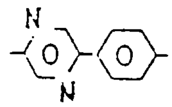 or 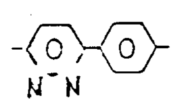

if $A^1$ is a single bond.

2. Chiral tilted smectic liquid-crystalline phase according to Claim 1, characterised in that it does not contain any chiral phenyl benzyl ethers.

3. Chiral tilted smectic liquid-crystalline phase according to Claim 1, characterised in that it contains less than 40% of chiral compounds.

4. Chiral tilted smectic liquid-crystalline phase according to Claim 1, characterised in that it contains one or two chiral compounds.

5. Chiral tilted smectic liquid-crystalline phase according to one of Claims 1 to 4, characterised in that it contains a liquid-crystalline component having negative dielectric anisotropy.

6. Phase according to Claim 5, characterised in that it contains at least one compound containing the structural element A, B or C

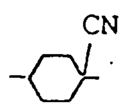 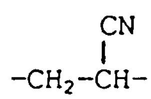 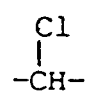

A B C

as the component having negative dielectric anisotropy.

7. Phase according to one of Claims 1 to 6, characterised in that it contains at least one compound of the formula II,

$R^4-A^1-COX-A^2-R^5$ II

in which

$R^4$ and $R^5$ are each alkyl having 1-15 C atoms, in which one or two non-adjacent $CH_2$ groups can also be replaced by -O-, -CO-, -O-CO-, -CO-O- and/or -CH=CH-,

X is O or S, and

$A^1$ and $A^2$ are each 1,4-phenylene or trans-1,4-cyclohexylene, one of the groups $A^1$ and $A^2$ is also, if desired, 4,4'-biphenylyl or trans,trans-4,4'-bicyclohexyl.

**8.** Phase according to one of Claims 1 to 7, characterised in that

$A^1$ is a single bond and

$A^2$ is

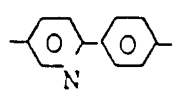 or 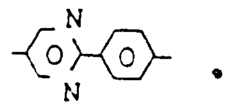

**9.** Phase according to Claim 8, characterised in that

$R^1$ is n-alkyl having 7 to 10 C atoms and

$R^2$ is n-alkoxy having 6 to 12 C atoms.

**10.** Phase according to Claim 9, characterised in that

$R^1$ is n-heptyl, n-octyl or n-nonyl.

**11.** Phase according to one of Claims 8 to 10, characterised in that the sum of C atoms of groups $R^1$ and $R^2$ is 15 or more.

**12.** Phase according to Claim 11, characterised in that the sum of C atoms of groups $R^1$ and $R^2$ is 15 to 20.

**13.** Phase according to one of Claims 8 to 12, characterised in that it contains only those compounds of the formula I according to Claim 1 in which $A^1$ is a single bond.

**14.** Phase according to one of Claims 1 to 13, characterised in that it contains at least one compound of the formulae

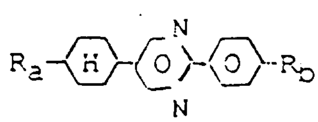 or 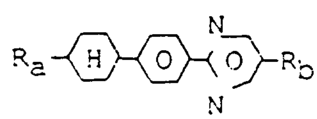

in which

$R_a$ is alkyl having 3 to 12 C atoms and

$R_b$ is alkyl with [sic] alkoxy each having 5 to 12 C atoms.

**15.** Phase according to one of Claims 1 to 14, characterised in that it contains at least one compound of the formula V,

$$R^1\text{-}Q^1\text{-}A\text{-}(Q^2)_q\text{-}R^2 \qquad V$$

in which

$R^1$ and $R^2$ are each, independently of one another, a straight-chain alkyl group having 1 to 15 C atoms, in which one or more non-adjacent $CH_2$ groups can also be replaced by -O-, -S-, -CO-, $-CHCH_3-O-$, $-CHCH_3-$, -CH-halogen-, -CHCN-, -O-CO-, -O-COO-, -CO-O- and/or -CH=CH-,

A is

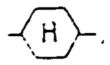, 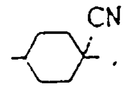, 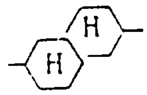,

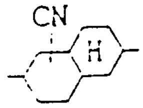, 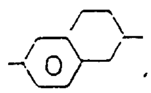, 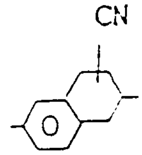,

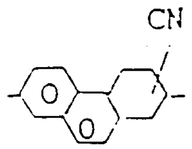, 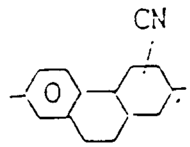

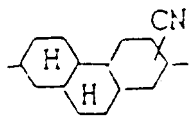, 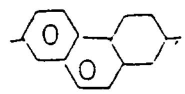

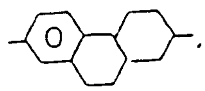, 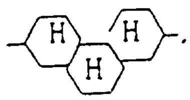,

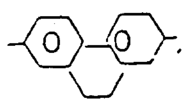, 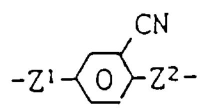 or

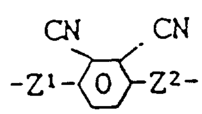

q — is 0 or 1,

$Q^1$ and $Q^2$ — are each, independently of one another, $-(A°-Z°)_p-$, in which

A° — is 1,4-cyclohexylene which is unsubstituted or mono- or polysubstituted by halogen atoms, $CH_3$ and/or nitrile groups and in which one or two non-adjacent $CH_2$ groups can also be replaced by -O- and/or -S- and/or a

$$-\overset{|}{C}H-CH_2-$$

grouping by

$$-\overset{|}{C}=N-$$

(Cy), or is 1,4-phenylene which is unsubstituted or mono- or polysubstituted by halogen atoms, $CH_3$ and/or nitrile groups and in which one or more CH groups can also be replaced by N (Ph), one of the radicals A° also [lacuna] 2,6-naphthylene (Na) or tetrahydro-2,6-naphthylene (4H-Na) each of which is unsubstituted or substituted by halogen or CN,

Z°, $Z^1$ and $Z^2$ — are each, independently of one another, -CO-O-, -O-CO-, $-CH_2O-$, $-OCH_2-$,

$-CH_2CH_2-$, $-CHCNCH_2-$, $-CH_2-CHCN-$ or a single bond, and

p is 1, 2 or 3, or, in the case where A is tetra- or octahydrophenanthrene, is also 0, at least one group Z° in the case where A is

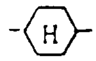, 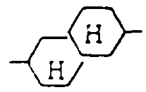, 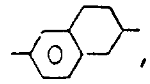,

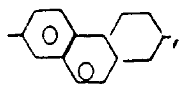, 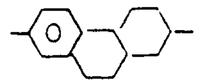,

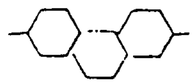 or 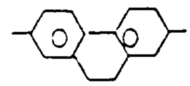,

being $-CHCNCH_2-$ or $-CH_2CHCN-$ and/or at least one $CH_2$ group being replaced in at least one of the groups $R^1$ and $R^2$ by -CHCN-.

**16.** Phase according to Claim 15, characterised in that achiral base mixtures composed of compounds of the formula V have been doped with chiral compounds of the formula I or else additionally with other chiral compounds.

**17.** Phase according to Claim 15, characterised in that it contains at least one compound selected from formulae V1 to V18:

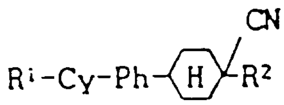

V1

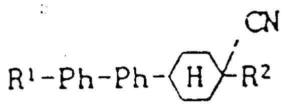

V2

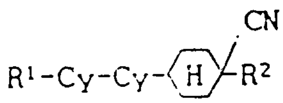

V3

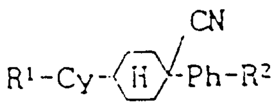

V4

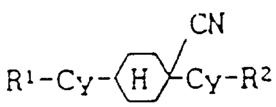

V5

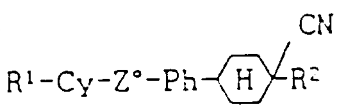

V6

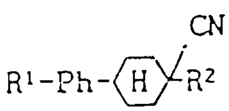

V7

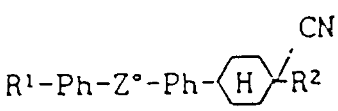

V8

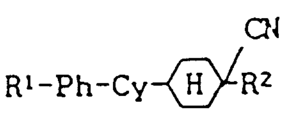

V9

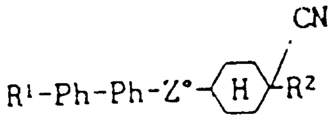
V10

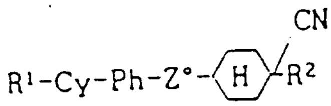
V11

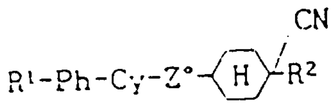
V12

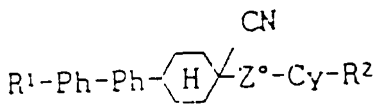
V13

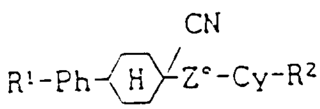
V14

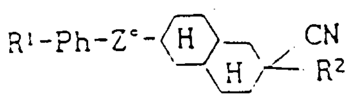
V15

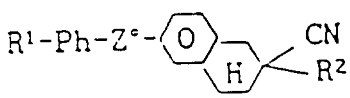
V16

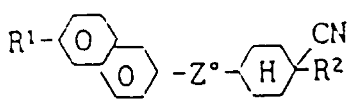
V17

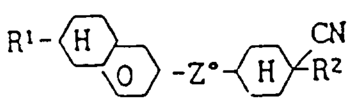
V18

in which $R^1$, $R^2$, Ph, Cy and Z° have the meaning given.

**18.** Phase according to Claim 15, characterised in that it contains at least one compound selected from formulae V19 to V22:

$R^1$-A°-Cy-$(CH_2)_r$-CHCN-$C_sH_{2s+1}$ V19

$R^1$-A°-A°-Cy-$(CH_2)_r$-CHCN-$C_sH_{2s+1}$ V20

$R^1$-A°-A°-CHCN-$CH_2$-Cy-$R^2$ V21

$R^1$-A°-A°-$CH_2$-CHCN-Cy-$R^2$ V22

in which r is 0, 1, 2 or 3 and (r + s) is 1 to 14.
Compounds of the formula I which do not exhibit $S_c$ phases are also suitable as components of smectic phases according to the invention.

**19.** Phase according to one of Claims 1 to 18, characterised in that it contains at least one compound of the formula IIIc

$R'-Q^3-Q^4-R'''$ IIIc

in which

| | |
|---|---|
| R | is straight-chain alkyl or alkoxy each having 2 to 10 C atoms, |
| $Q^3$ and $Q^4$ | are each 1,4-phenylene, 4,4'-biphenylene, or trans-1,4-cyclohexylene, one of the groups $Q^3$ and $Q^4$ is also 1,4-phenylene in which at least one CH group is replaced by N, and |
| R''' | is an optically active radical having an asymmetric carbon atom of the structure |

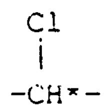 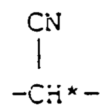 .

**20.** Phase according to Claim 21 [sic], characterised in that it contains a compound of the formula IIIc',

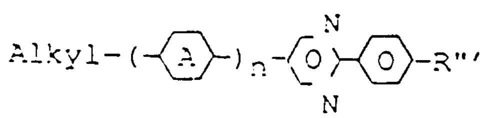 IIIc'

in which

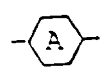

is 1,4-phenylene or trans-1,4-cyclohexylene, and n is 0 or 1.

**21.** Electro-optical display element, characterised in that it contains a phase according to one of Claims 1 to 20 as the dielectric.

**Revendications**
**Revendications pour les Etats contractants suivants : CH, DE, FR, GB, LI**

**1.** Phase à cristaux liquides smectique inclinée chirale, caractérisée en ce qu'elle contient au moins trois composés de formule I

$R^1-A^1-A^2-R^2$ I

dans laquelle

| | |
|---|---|
| $R^1$ et $R^2$ | représentent chacun un groupe alkyle en C 1-C 15 dans lequel un ou deux groupes $CH_2$ non voisins peuvent être remplacés par -O-, -S-, -CO-, -O-CO-, -CO-O-, -CO-S-, -S-CO-, -CHhalogène-, -CHCN- et/ou -CH = CH-, |
| $A^1$ | représente un groupe 1,4-phénylène, trans-1,4-cyclohexylène ou une liaison simple |
| $A^2$ | représente |

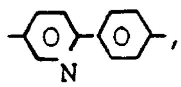, 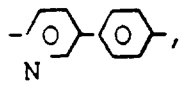, 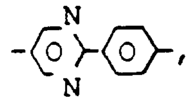, 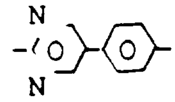

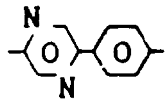, 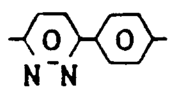, 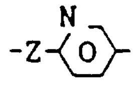, 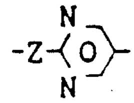,

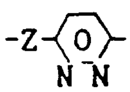 ou 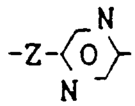

dans lesquels Z représente -O-CO-, -CO-O-, -S-CO-, -CO-S-, $-CH_2O-$, $-OCH_2-$ ou $-CH_2CH_2-$ sous réserve que

a) $A^2$ représente

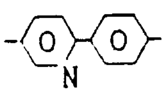, 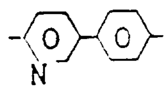, 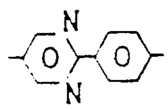, 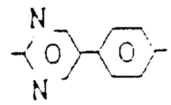

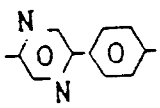 ou 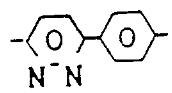

lorsque $A^1$ représente une liaison simple, et

b) lorsque $A^1$ représente une liaison simple et que $A^2$ représente

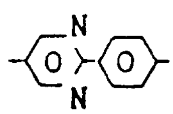

les phases contenant des composés de formule

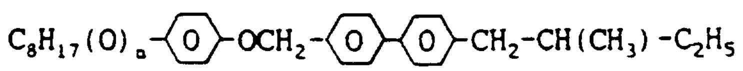

dans laquelle n est égal à 0 ou 1, sont exclues.

2. Phase à cristaux liquides smectique inclinée chirale selon revendication 1, caractérisée en ce qu'elle ne contient pas d'éther phénylbenzylique chiral.

3. Phase à cristaux liquides smectique inclinée chirale selon revendication 1, caractérisée en ce qu'elle contient moins de 40 % de composés chiraux.

**4.** Phase à cristaux liquides smectique inclinée chirale selon revendication 1, caractérisée en ce qu'elle contient un ou deux composés chiraux.

**5.** Phase à cristaux liquides smectique inclinée chirale selon une des revendications 1 à 4, caractérisée en ce qu'elle contient un composant à cristaux liquides à anisotropie diélectrique négative.

**6.** Phase selon revendication 5, caractérisée en ce qu'elle contient en tant que composant à anisotropie diélectrique négative au moins un composé contenant l'élément de structure A, B ou C

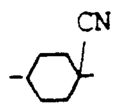 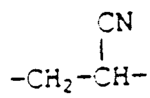 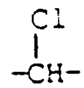

A B C

**7.** Phase selon l'une des revendications 1 à 6, caractérisée en ce qu'elle contient au moins un composé de formule II

$R^4$-$A^1$-COX-$A^2$-$R^5$ II

dans laquelle

$R^4$ et $R^5$ représentent chacun un groupe alkyle en C 1-C 15 dans lequel un ou deux groupes $CH_2$ non voisins peuvent être remplacés par -O-, -CO-, -O-CO-, -CO-O-et/ou -CH = CH-,

X représente O ou S, et

$A^1$ et $A^2$ représentent chacun un groupe 1,4-phénylène ou trans-1,4-cyclohexylène, l'un des symboles $A^1$ et $A^2$ pouvant éventuellement représenter également un groupe 4,4'-biphénylyle ou trans,trans-4,4'-bicyclohexyle.

**8.** Phase selon l'une des revendications 1 à 7, caractérisée en ce que :

$A^1$ représente une liaison simple et $A^2$ représente

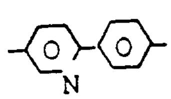 ou 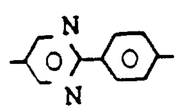

**9.** Phase selon revendication 8, caractérisée en ce que :

$R^1$ représente un groupe n-alkyle en C 7-C 10, et

$R^2$ représente un groupe n-alcoxy en C 6-C 12.

**10.** Phase selon revendication 9, caractérisée en ce que :

$R^1$ représente un groupe n-heptyle, n-octyle ou n-nonyle.

**11.** Phase selon l'une des revendications 8 à 10, caractérisée en ce que la somme des atomes de carbone des groupes $R^1$ et $R^2$ est de 15 ou plus.

**12.** Phase selon revendication 11, caractérisée en ce que la somme des atomes de carbone des groupes $R^1$ et $R^2$ est de 15 à 20.

**13.** Phase selon l'une des revendications 8 à 12, caractérisée en ce qu'elle contient uniquement des composés de formule I de la revendication 1 dans laquelle $A^1$ représente une liaison simple.

**14.** Phase selon l'une des revendications 1 à 13, caractérisée en ce qu'elle contient au moins un composé de formule

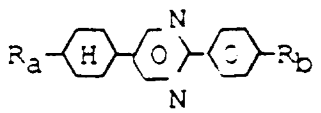 ou 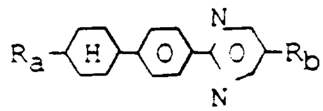

dans laquelle

$R_a$ représente un groupe alkyle en C 3-C 12, et

$R_b$ représente un groupe alkyle ou alcoxy contenant chacun 5 à 12 atomes de carbone.

**15.** Phase selon l'une des revendications 1 à 14, caractérisée en ce qu'elle contient au moins un composé de formule V

$$R^1\text{-}Q^1\text{-}A\text{-}(Q^2)_q\text{-}R^2 \qquad V$$

dans laquelle

$R^1$ et $R^2$ représentent chacun, indépendamment l'un de l'autre, un groupe alkyle à chaîne droite en C 1-C 15, dans lequel un ou plusieurs groupes $CH_2$ non voisins peuvent être remplacés par -O-, -S-, -CO-, $-CHCH_3-O-$, $-CHCH_3-$, -CH-halogène, -CHCN-, -O-CO-, -O-COO-, -CO-C- et/ou -CH=CH-,

A représente

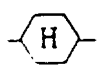, 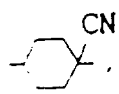, 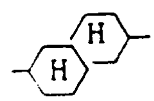,

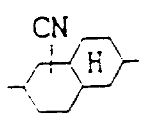, 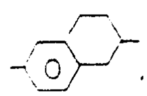, 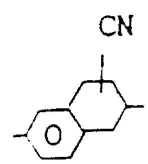,

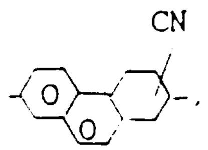
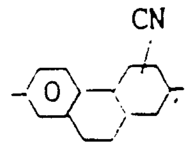

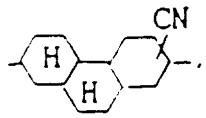
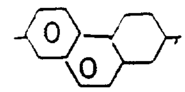

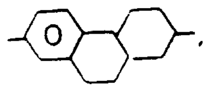
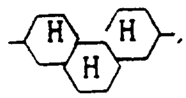

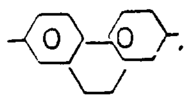
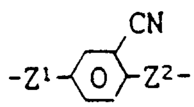 ou

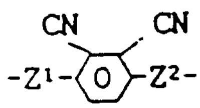

q est égal à 0 ou 1,

$Q^1$ et $Q^2$ représentent chacun, indépendamment l'un de l'autre $-(A°-Z°)_p-$ dans lequel

A° représente un groupe 1,4-cyclohexylène non substitué ou substitué une ou plusieurs fois par des atomes d'halogènes, des groupes $CH_3$ et/ou nitrile et dans lequel un ou deux groupes $CH_2$ non voisins peuvent être remplacés par -O- et/ou -S- et/ou un groupement

$$-\overset{|}{C}H-CH_2-$$

peut être remplacé par

$$-\overset{|}{C}=N-\ (Cy),$$

ou un groupe 1,4-phénylène non substitué ou substitué une ou plusieurs fois par des atomes d'halogènes, des groupes $CH_3$ et/ou nitrile et dans lequel un ou plusieurs groupes CH peuvent être remplacés par N (Ph), l'un des symboles A° pouvant également représenter un groupe 2,6-naphtylène (Na) ou tétrahydro-2,6-naphtylène (4H-Na), éventuellement substitué par un halogène ou un groupe CN,

Z°, $Z^1$ et $Z^2$ représentent chacun, indépendamment les uns des autres, -CO-O-, -O-CO-, $-CH_2O-$, $-OCH_2-$, $-CH_2CH_2-$, $-CHCNCH_2-$, $-CH_2CHCN-$ ou une liaison simple, et

p est égal à 1, 2 ou 3 ou bien encore, lorsque A représente un cycle tétra- ou octa-hydrophénanthrène, à O, étant précisé que lorsque A représente

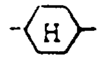, 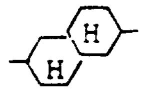, 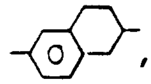,

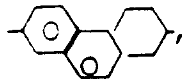, 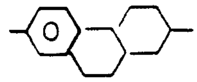,

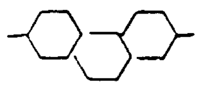 oder 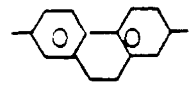,

au moins un symbole Z° représente -CHCNCH$_2$- ou -CH$_2$CHCN- et/ou dans au moins un des groupes $R^1$ et $R^2$, au moins un groupe $CH_2$ est remplacé par -CHCN-.

**16.** Phase selon revendication 15, caractérisée en ce que les mélanges de base achiraux de composés de formule V sont dopés par des composés chiraux de formule I ou encore par d'autres composés chiraux.

**17.** Phase selon revendication 15, caractérisée en ce qu'elle contient au moins un composé choisi parmi les composés de formules V1 à V18 :

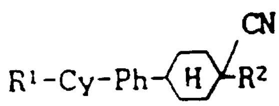 V1

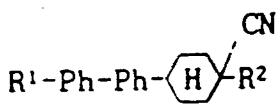 V2

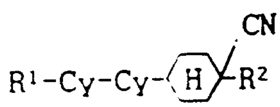 V3

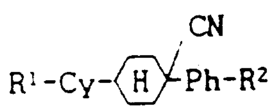 V4

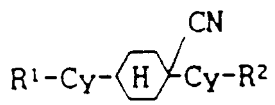

V5

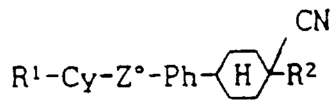

V6

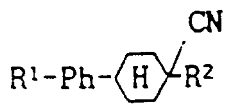

V7

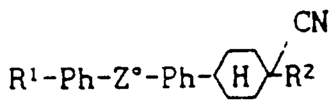

V8

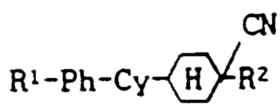

V9

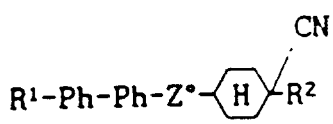

V10

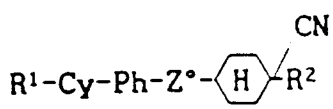

V11

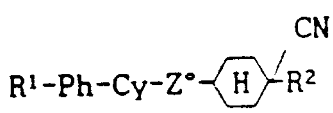

V12

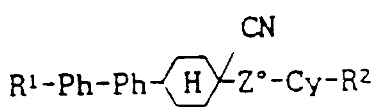

V13

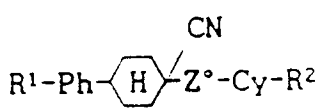

V14

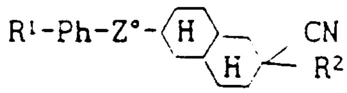 V15

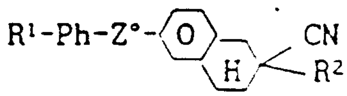 V16

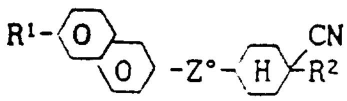 V17

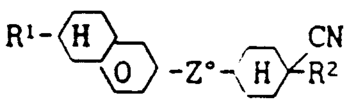 V18

dans lesquelles $R^1$, $R^2$, Ph, Cy et Z° ont les significations indiquées ci-dessus.

**18.** Phase selon revendication 15, caractérisée en ce qu'elle contient au moins un composé choisi parmi les composés de formules V19 à V22 :

$R^1$-A°-Cy-$(CH_2)_r$-CHCN-$C_sH_{2s+1}$ V19

$R^1$-A°-A°-Cy-$(CH_2)_r$-CHCN-$C_sH_{2s+1}$ V20

$R^1$-A°-A°-CHCN-$CH_2$-Cy-$R^2$ V21

$R^1$-A°-A°-$CH_2$-CHCN-Cy-$R^2$ V22

dans lesquelles r est égal à 0, 1, 2 ou 3 et (r + s) a une valeur de 1 à 14.

Les composés de formule I n'ayant pas de phase $S_c$ conviennent également en tant que composants des phases smectiques selon l'invention.

**19.** Phase selon l'une des revendications 1 à 18, caractérisée en ce qu'elle contient un composé de formule IIIc

R'-$Q^3$-$Q^4$-R''' IIIc

dans laquelle

| | |
|---|---|
| R | représente un groupe alkyle ou alcoxy à chaîne droite contenant chacun 2 à 10 atomes de carbone, |
| $Q^3$ et $Q^4$ | représentent chacun un groupe 1-4-phénylène, 4,4'-biphénylène ou trans-1,4-cyclohexylène, l'un des symboles $Q^3$ et $Q^4$ pouvant également représenter un groupe 1,4-phénylène dans lequel au moins un groupe CH est remplacé par N, et |
| R''' | représente un radical possédant l'activité optique, contenant un atome de carbone asymétrique, de structure |

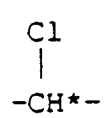 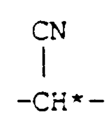

**20.** Phase selon revendication 21, caractérisée en ce qu'elle contient un composé de formule IIIc'

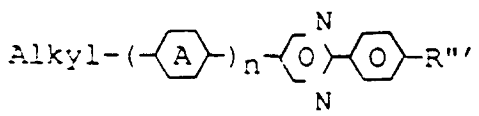 IIIc'

dans laquelle

représente un groupe 1,4-phénylène ou trans-1,4-cyclohexylène, et

n est égal à 0 ou 1.

**21.** Elément d'affichage électro-optique, caractérisé en ce qu'il contient en tant que diélectrique une phase selon l'une des revendications 1 à 20.

**Revendications pour les Etats contractants suivants : AT, BE, IT, NL, SE**

**1.** Phase à cristaux liquides smectique inclinée chirale, caractérisée en ce qu'elle contient au moins trois composés de formule I

$R^1$-$A^1$-$A^2$-$R^2$ I

dans laquelle

- $R^1$ et $R^2$ représentent chacun un groupe alkyle en C 1-C 15 dans lequel un ou deux groupes $CH_2$ non voisins peuvent être remplacés par -O-, -S-, -CO-, -O-CO-, -CO-O-, -CO-S-, -S-CO-, -CHhalogène-, -CHCN- et/ou -CH = CH-,
- $A^1$ représente un groupe 1,4-phénylène, trans-1,4-cyclohexylène ou une liaison simple
- $A^2$ représente

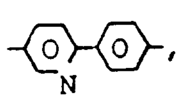, 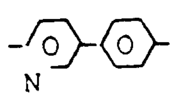, 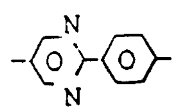, 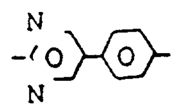

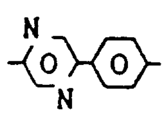, 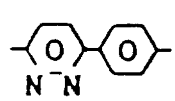, 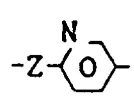, 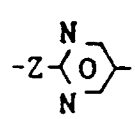,

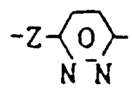 ou 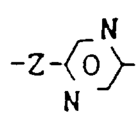

dans lesquels Z représente -O-CO-, -CO-O-, -S-CO-, -CO-S-, $-CH_2O-$, $-OCH_2-$ ou $-CH_2CH_2-$ sous réserve que $A^2$ représente

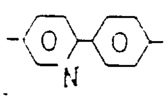, 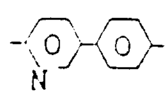, 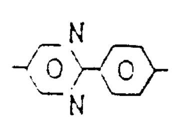, 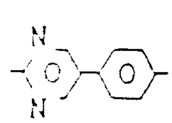

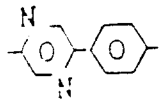 ou 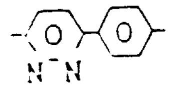

lorsque $A^1$ représente une liaison simple.

**2.** Phase à cristaux liquides smectique inclinée chirale selon revendication 1, caractérisée en ce qu'elle ne contient pas d'éther phénylbenzylique chiral.

**3.** Phase à cristaux liquides smectique inclinée chirale selon revendication 1, caractérisée en ce qu'elle contient moins de 40 % de composés chiraux.

**4.** Phase à cristaux liquides smectique inclinée chirale selon revendication 1, caractérisée en ce qu'elle contient un ou deux composés chiraux.

**5.** Phase à cristaux liquides smectique inclinée chirale selon une des revendications 1 à 4, caractérisée en ce qu'elle contient un composant à cristaux liquides à anisotropie diélectrique négative.

**6.** Phase selon revendication 5, caractérisée en ce qu'elle contient en tant que composant à anisotropie diélectrique négative au moins un composé contenant l'élément de structure A, B ou C

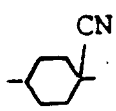 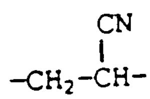 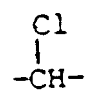

A B C

**7.** Phase selon l'une des revendications 1 à 6, caractérisée en ce qu'elle contient au moins un composé de formule II

$R^4$-$A^1$-COX-$A^2$-$R^5$ II

dans laquelle

| | |
|---|---|
| $R^4$ et $R^5$ | représentent chacun un groupe alkyle en C 1-C 15 dans lequel un ou deux groupes $CH_2$ non voisins peuvent être remplacés par -O-, -CO-, -O-CO-, -CO-O- et/ou -CH=CH-, |
| X | représente O ou S, et |
| $A^1$ et $A^2$ | représentent chacun un groupe 1,4-phénylène ou trans-1,4-cyclohexylène, l'un des symboles $A^1$ et $A^2$ pouvant éventuellement représenter également un groupe 4,4'-biphénylyle ou trans,trans-4,4'-bicyclohexyle. |

**8.** Phase selon l'une des revendications 1 à 7, caractérisée en ce que :

$A^1$ représente une liaison simple et $A^2$ représente

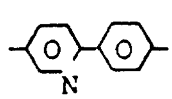 ou 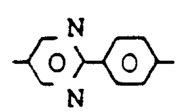

**9.** Phase selon revendication 8, caractérisée en ce que :

$R^1$ représente un groupe n-alkyle en C 7-C 10, et

$R^2$ représente un groupe n-alcoxy en C 6-C 12.

**10.** Phase selon revendication 9, caractérisée en ce que :
$R^1$ représente un groupe n-heptyle, n-octyle ou n-nonyle.

**11.** Phase selon l'une des revendications 8 à 10, caractérisée en ce que la somme des atomes de carbone des groupes $R^1$ et $R^2$ est de 15 ou plus.

**12.** Phase selon revendication 11, caractérisée en ce que la somme des atomes de carbone des groupes $R^1$ et $R^2$ est de 15 à 20.

**13.** Phase selon l'une des revendications 8 à 12, caractérisée en ce qu'elle contient uniquement des composés de formule I de la revendication 1 dans laquelle $A^1$ représente une liaison simple.

**14.** Phase selon l'une des revendications 1 à 13, caractérisée en ce qu'elle contient au moins un composé de formule

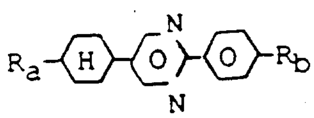 ou 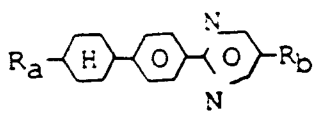

dans laquelle
$R_a$ représente un groupe alkyle en C 3-C 12, et
$R_b$ représente un groupe alkyle ou alcoxy contenant chacun 5 à 12 atomes de carbone.

**15.** Phase selon l'une des revendications 1 à 14, caractérisée en ce qu'elle contient au moins un composé de formule V

$R^1$-$Q^1$-A-$(Q^2)_q$-$R^2$ V

dans laquelle
$R^1$ et $R^2$ représentent chacun, indépendamment l'un de l'autre, un groupe alkyle à chaîne droite en C 1-C 15, dans lequel un ou plusieurs groupes $CH_2$ non voisins peuvent être remplacés par -O-, -S-, -CO-, $-CHCH_3-O-$, $-CHCH_3-$, -CH-halogène, -CHCN-, -O-CO-, -O-COO-, -CO-O- et/ou -CH=CH-,
A représente

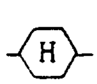, 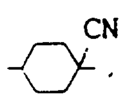, 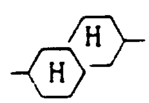,

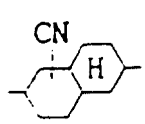, 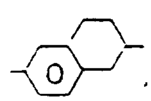, 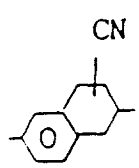,

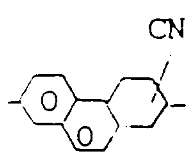, 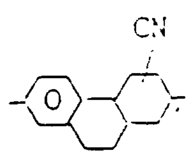

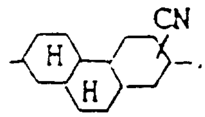 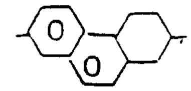

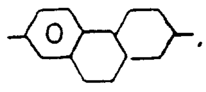 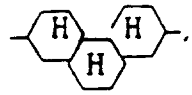

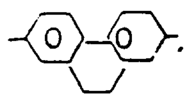 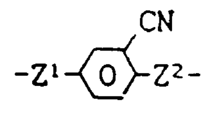 ou

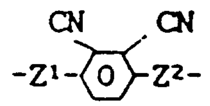

q est égal à 0 ou 1,

$Q^1$ et $Q^2$ représentent chacun, indépendamment l'un de l'autre $-(A°-Z°)_p-$ dans lequel

A° représente un groupe 1,4-cyclohexylène non substitué ou substitué une ou plusieurs fois par des atomes d'halogènes, des groupes $CH_3$ et/ou nitrile et dans lequel un ou deux groupes $CH_2$ non voisins peuvent être remplacés par -O- et/ou -S- et/ou un groupement

$$-\overset{|}{C}H-CH_2-$$

peut être remplacé par

$$-\overset{|}{C}=N-$$

(Cy), ou un groupe 1,4-phénylène non substitué ou substitué une ou plusieurs fois par des atomes d'halogènes, des groupes $CH_3$ et/ou nitrile et dans lequel un ou plusieurs groupes CH peuvent être remplacés par N (Ph), l'un des symboles A° pouvant également représenter un groupe 2,6-naphtylène (Na) ou tétrahydro-2,6-naphtylène (4H-Na), éventuellement substitué par un halogène ou un groupe CN,

Z°, $Z^1$ et $Z^2$ représentent chacun, indépendamment les uns des autres, -CO-O-, -O-CO-, $-CH_2O-$, $-OCH_2-$, $-CH_2CH_2-$, $-CHCNCH_2-$, $-CH_2-CHCN-$ ou une liaison simple, et

p est égal à 1, 2 ou 3 ou bien encore, lorsque A représente un cycle tétra- ou octa-hydrophénanthrène, à O, étant précisé que lorsque A représente

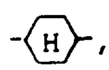, 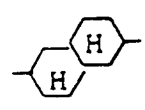, 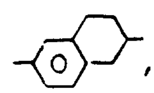,

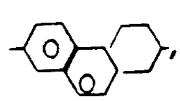, 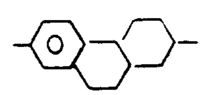,

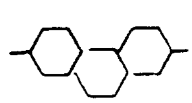 ou 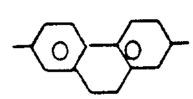,

au moins un symbole Z° représente $-CHCNCH_2-$ ou $-CH_2CHCN-$et/ou dans au moins un des groupes $R^1$ et $R^2$, au moins un groupe $CH_2$ est remplacé par -CHCN-.

**16.** Phase selon revendication 15, caractérisée en ce que les mélanges de base achiraux de composés de formule V sont dopés par des composés chiraux de formule I ou encore par d'autres composés chiraux.

**17.** Phase selon revendication 15, caractérisée en ce qu'elle contient au moins un composé choisi parmi les composés de formules V1 à V18 :

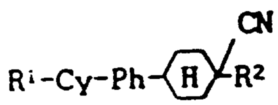

V1

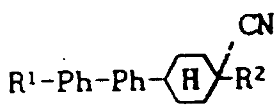

V2

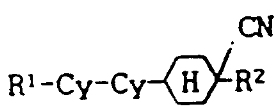

V3

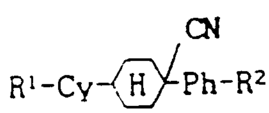

V4

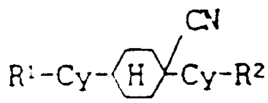

V5

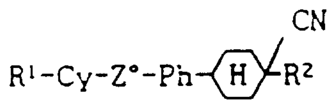

V6

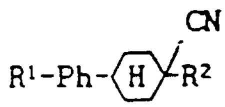

V7

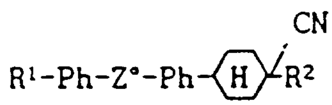

V8

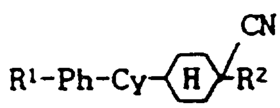

V9

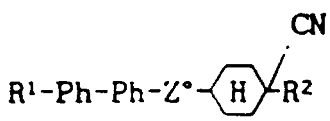

V10

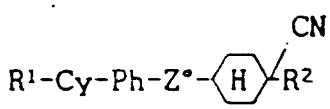

V11

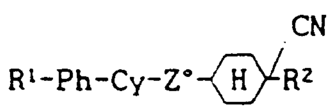

V12

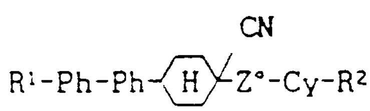

V13

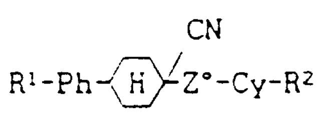

V14

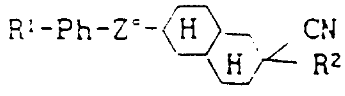 V15

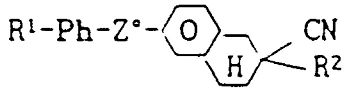 V16

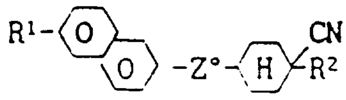 V17

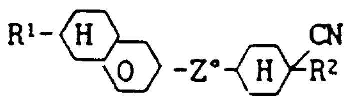 V18

dans lesquelles $R^1$, $R^2$, Ph, Cy et Z° ont les significations indiquées ci-dessus.

**18.** Phase selon revendication 15, caractérisée en ce qu'elle contient au moins un composé choisi parmi les composés de formules V19 à V22 :

$R^1$-A°-Cy-$(CH_2)_r$-CHCN-$C_sH_{2s+1}$ V19

$R^1$-A°-A°-Cy-$(CH_2)_r$-CHCN-$C_sH_{2s+1}$ V20

$R^1$-A°-A°-CHCN-$CH_2$-Cy-$R^2$ V21

$R^1$-A°-A°-$CH_2$-CHCN-Cy-$R^2$ V22

dans lesquelles r est égal à 0, 1, 2 ou 3 et (r + s) a une valeur de 1 à 14.

Les composés de formule I n'ayant pas de phase $S_c$ conviennent également en tant que composants des phases smectiques selon l'invention.

**19.** Phase selon l'une des revendications 1 à 18, caractérisée en ce qu'elle contient un composé de formule IIIc

R'-$Q^3$-$Q^4$-R''' IIIc

dans laquelle

- R représente un groupe alkyle ou alcoxy à chaîne droite contenant chacun 2 à 10 atomes de carbone,
- $Q^3$ et $Q^4$ représentent chacun un groupe 1,4-phénylène, 4,4'-biphénylène ou trans-1,4-cyclohexylène, l'un des symboles $Q^3$ et $Q^4$ pouvant également représenter un groupe 1,4-phénylène dans lequel au moins un groupe CH est remplacé par N, et
- R''' représente un radical possédant l'activité optique, contenant un atome de carbone asymétrique, de structure

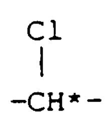 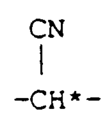

**20.** Phase selon revendication 21, caractérisée en ce qu'elle contient un composé de formule IIIc'

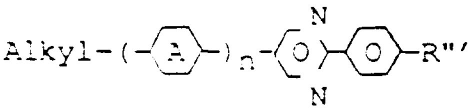

IIIc'

dans laquelle

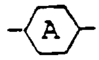

représente un groupe 1,4-phénylène ou trans-1,4-cyclohexylène, et

n est égal à 0 ou 1.

**21.** Elément d'affichage électro-optique, caractérisé en ce qu'il contient en tant que diélectrique une phase selon l'une des revendications 1 à 20.